# EUROPEAN PATENT SPECIFICATION

(11) **EP 2 451 845 B1**
(45) Date of publication and mention of the grant of the patent: **01.03.2017**
(21) Application number: 10796821.6
(22) Date of filing: 07.07.2010
(51) Int. Cl.: C08B 37/08, A23B 7/154

(54) **PRESERVATIVES FROM CHITIN DERIVATIVES**
PRESERVATIVE AUS CHITINDERIVATEN
CONSERVATEURS ISSUS DE DÉRIVÉS DE CHITINE

(30) Priority: 07.07.2009 IN MU16052009; 28.05.2010 IN MU16432010
(43) Date of publication of application: 16.05.2012
(73) Proprietor: Camlin Fine Sciences Limited, Mumbai 400 093 (IN)
(72) Inventor: SHENDYE, Abhay Parashuram, Maharashtra (IN)
(74) Representative: advotec.
(86) International application number: PCT/IN2010/000458
(87) International publication number: WO 2011/004398

(56) References cited:
- WO-A1-87/07618
- WO-A1-03/070008
- CN-A- 1 709 166
- JP-A- 8 157 501
- JP-A- 8 157 501
- KR-B1- 100 262 253
- US-A- 2 783 148
- US-A- 5 061 792
- US-A- 5 597 811
- US-A1- 2007 077 305
- US-A1- 2007 104 836
- US-A1- 2007 181 038
- XU J. ET AL: 'Chitosan Film Acylation and Effects on Biodegradability' MACROMOLECULES vol. 29, 1996, pages 3436 - 3440, XP000583951
- MOORE G.K. ET AL: 'Studies on the acetylation of chitosan' PROCEEDINGS OF THE FIRST INTERNATIONAL CONFERENCE ON CHITIN/CHITOSAN 1978, pages 421 - 429, XP008149128
- AGNIHOTRI S.A ET AL: 'Recent advances on chitosan-based micro- and nanoparticles in drug delivery' JOURNAL OF CONTROLLED RELEASE vol. 100, 2004, pages 5 - 28, XP004604081

## Description

### TECHNICAL FIELD

The invention relates to compositions, process for production of compositions of chitosan that act as a respiration controller, anti-microbial, microbiostatic, efficient remover of microbes and anti-oxidative for keeping freshness of fruits, vegetables, cut flowers, meat, fish and other processed food products and methods / processes of applying the same and the applicators for the same.

### BACKGROUND OF THE INVENTION

Chitosan has been used in the past as post harvest preservatives of fresh fruits and vegetables. The chitosan used is isolated from prawn shell, crab shell, daphnia shell, squid, or any other crustacean, fungi, and insect skin; and treated for varying degree of acetylation and dissolved in 1 - 2 % acetic acid.

Chitosan has been used for improving the shelf life of various fruits and vegetables such as citrus [Magnetic Resonance Imaging 22 (2004) 27 - 37], carrot [Food Control 17 (2006) 336 - 41], lettuce and strawberry [Food Microbiology 21(2004)703-14], and litchi [Postharvest Biology and Technology 38 (2005) 128 - 36] wherein the chitosan used is of more than 43 KD (Kilo Dalton) size and 93% de-acetylated. Anti-oxidant property of chitosan has been studied on Salmon [Food Chem 101 (2007) 308-11] where chitosan of three different molecular weights has been used. Solution (1%) of 30 kD chitosan showed results equivalent to chemical anti-oxidant BHT, and were superior to those obtained using chitosan of 90 kD and 120 kD molecular weight. Useful preservation activity of chitosan obtained from prawn shell has been reported for various foods such as eggs, meat, sausage, sea food, tofu, Juices, Mayonnaise, Kimchi, Noodles, Rice cake, soy bean sprouts, starch jelly etc. In case of milk preservation chitosan of molecular weight varying from 0.2 - 30 kD has been described [Korean J. Food Sci. Tech. 32 (2000) 806 - 13]. In case of bread three different molecular weights of chitosan - 2 kD [J Chitin Chitosan 7 (2002) 214 - 8], 120 kD [Korean J Food Nutrition 11 (2003) 309 - 15] and 493 kD [J Chitin Chitosan 7 (2002) 208 - 13] have been used successfully for extending its shelf life, sustaining weight, and retarding starch degradation. Antimicrobial activity of chitosan has been reported against various bacteria yeasts and molds such as Aeromonas, Bacillus, Bifidobacterium, brochothrix, Clostridium, Enterococcus, Escerechia, Lactobacillus, Leuconostoc, Listeria, Micrococcus, Pediococcus, Photobacterium, Pseudomonas, Salmonella, Shigella, Staphylococcus, Vibrio, Candida, Cryptococcus, Saccharomyces, Schizosaccaromyces, Zygosaccharomyces, Aspergillus, Botrytis, Cladosporium, Penicillium, Rhizopus, etc. and has been reviewed [J Food Sci 72 (2007) R 87 - R100]. Except for the few examples mentioned specifically, all prior art methods have used the high molecular weight chitosan obtained from prawn shell with varying and undefined molecular weights and degree of deacetylation.

US patent no. 5,374,627 describes method of control of plant diseases and damage by certain pests in agricultural plants by compositions containing 1 part by weight of a chitosan hydrolyzate with an average molecular weight of 10,000 to 50,000, obtained by acid hydrolysis or enzymatic hydrolysis of chitosan.

The patent application WO 03070008 discloses antimicrobial compounds and their methods of application wherein the product for use is a liquid obtained by using organic acids of 3 - 30 carbons and the claimed product is useful in the preservation of the acidic foods only. In one of the example chitosanase enzyme is used for degradation of chitosan.

Although various chemical modifications of chitosan are known in the literature that lead to solubilization of chitosan at neutral to alkaline pH, the applications of chitosan in food are only using solution of chitosan in organic acid preferably acetic acid. The solution thus obtained is of low pH (2.0 - 4.0) depending on the concentration of the acid used. The acid used in the said formulation adds taste to the product to be treated, and/or adversely affects the shelf life. In case of banana, acetic acid is known to develop senescent spots [black discolouration of the skin] which is undesired. Acids used in the chitosan formulation are responsible for curdling of the milk and milk products. The net positive charge developed on the chitosan molecule because of the low pH is responsible for separation of anionic components from the product to be treated [such as inorganic salts, fatty acids], which adsorb on the positively charged chitosan. All these reactions are undesired and can be avoided by making the formulation as described in the present invention.

A class of water soluble chitosan as described in the literature is of complexes of chitosan with respective organic acids which are typically described as chitosan acetate, chitosan lactate etc. These are dry powders of chitosan acid complex, derived by simple mixing followed by removal of un-reacted acid and drying. Such complexes are of poor chemical definition, rather unstable, and still have the problems associated with low pH like that of the chitosan solution.

The partial degradation products of chitosan like chito-oligosaccharides are known to be water soluble. However, these molecules have varying degree of biological activities such as respiration control, microbio-stasis, coating characteristics etc. These are generally very weak in the desired levels of activities as compared with the products developed using the method(s) claimed in the present invention.

### SUMMARY OF THE INVENTION

The invention comprises a process for producing a chitosan derivative, and a product prepared thereby, by activating chitosan by suspending the said chitosan in alcohol that contains water from 0% to maximum of about 30%, stirring with heating for a period of time to get activated chitosan and there after reacting the said activated chitosan with a reagent capable of complexing by physical forces or chemical forces to prepare a derivative. Chitosan derivatives disclosed by this invention and method of preparing them results in derivatives have a range of properties that comprise, without limitation, an antimicrobial, or a microbiostatic, or an antitranspirant, or an antifungal, or a fruit preservative properties or a combination thereof. The said physical forces include adsorption and the said chemical forces include a covalent bond.

About 8 embodiments of process and resultant products of this invention are illustrated that are described as Second and fourth to Tenth Composition and processes of making them. Products of this invention can be further derivatized to modify or to add to properties of each of them to cover broader or different scope of application.

Adding chitosan to alcohol, adding water about 20% of volume of alcohol to prepare a mixture and further adding Lactic acid or any other inorganic acid or any other organic acid to the said mixture, stirring at a temperature of about below 30°C, more preferably at around 25°C leads to formation of a First Composition in suspension, which is an acid derivative that can be used as such or after drying or derivatized further.

Adding chitosan to alcohol, adding water about 20% of volume of alcohol to prepare a mixture and heating the said mixture to about 70°C, adding Succinic anhydride to this mixture, stirring and holding at a temperature between to about 60 - 70°C and preferably at about 65°C leads to formation of a Second Composition in suspension,

Preparing First Composition and succinylating the same provides Third Composition. Thus, adding chitosan to alcohol, adding water about 20% of volume of alcohol to prepare a mixture and adding Lactic acid or any other inorganic acid or any other organic acid to the said mixture at a rate of about fifth or one tenth of the weight of the raw material taken and stirring 2 hours or for a period of time enough for reaction maintaining a temperature below 30°C, more preferably at around 25°C, to get a lactic or another acid derivative in suspension, isolating the lactic / acid derivative, subjecting the isolated derivative after filtering, to further reaction by suspending the same in water:alcohol mixture, preferably a 20:80 mixture, adding succinic anhydride taken by weight upto one tenth of the dry weight of the said lactic / another acid derivative to get a Third Composition in suspension that can be isolated.

Making Second Composition and making its acid derivative with an acid, preferably with lactic acid, provides Fourth Composition of this invention. Thus, adding chitosan to alcohol, adding water about 20% of volume of alcohol to prepare a mixture and heating the said mixture to about 70°C, adding succinic anhydride at a rate of about 10% by weight of the raw material to this mixture, stirring for 1 hour or for a period of time enough for reaction maintaining a temperature to about 60 - 70°C and preferably at about 65°C to get a succinic derivative in suspension, allowing the suspension to cool to about 30°C, subjecting the said succinic derivative to further reaction with lactic or another acid about 10% to 20% of the dry weight of the succinic derivative, stirring the mixture for about 1 hour, filtering and drying the solids to get a Fourth Composition in suspension that can be isolated.

Preparing Second composition, deaceylating the same with ENZYME xylan acyl transferase or any other aceylating enzyme for a period of time, preferably about 6 to 7 hours, followed by chitinolysis by a chitinolytic enzyme or preferably a proteolytic as well as chitinolytic enzyme that further preferably is thermostable, and further preferably produced from *Aspergillus niger* MTCC 5572 (deposited at The Microbial Type Culture Collection (MTCC), Institute of Microbial Technology, Chandigarh, India) for a period of time, preferably for about one hour, provides Fifth Composition of this invention in suspension that can be recovered. Thus, suspending chitosan in water: alcohol mixture 20:80, mixing the suspension well by stirring preferably at 100 rpm for a period enough to get good activation preferably for 30 minutes, heating and maintaining the said mixture to about 70°C, adding Succinic anhydride to this mixture, stirring for 1 hour or for a period of time enough for reaction maintaining the temperature between 60 about - 70°C and preferably at about 65°C for a period of time, allowing the suspension to cool to get a succinic derivative, subjecting the said succinic derivative to further reaction with Lactic or another acid about 10% weight of the dry weight of the succinic derivative, for every 5 gram dry weight of succinic derivative used adding enzyme for deaceylation about 4000 I.U, stirring the mixture for a period of time, preferably for 7 hours, further adding a chitinolytic enzyme, preferably around 500 I.U. per 5 g dry weight of succinic derivative, continuing stirring for a period of time, preferably for 1 hour, allowing the suspension to cool provides a Fifth Composition in suspension, that may be recovered and used.

Reacting Fifth Composition in alcohol with sugar and then with formaldehyde provides Sixth Composition of this invention. Thus adding chitosan to alcohol, adding water about 20% of volume of alcohol to prepare a mixture, heating and maintaining the said mixture to about 70°C, adding Succinic anhydride to this mixture, stirring for 1 hour or for a period of time enough for reaction maintaining the temperature between 60 about - 70°C and preferably at about 65°C for a period of time, allowing the suspension to cool to get a succinic derivative, subjecting the said succinic derivative to further reaction with Lactic or another acid about 10% weight of the dry weight of the succinic derivative, per 5 gram dry weight of succinic derivative used adding enzyme for deaceylation about 4000 I.U, stirring the mixture for a period of time, preferably for 7 hours, further adding a chitinolytic enzyme, preferably around 500 I.U. per 5 g dry weight of succinic derivative, continuing stirring for a period of time, preferably for 1 hour, allowing the suspension to cool to get a Fifth Composition in suspension, suspending the dry powder of the said Fifth Composition in absolute alcohol, mixing the suspension well by stirring at 100 rpm for 30 min. and heating and maintaining the temperature throughout the process to 60°C, adding sugar dissolved in water in about 10% volume to volume of the alcohol, stirring the mixture for about 15 minutes or for a period of time enough for mixing, adding formaldehyde dropwise over about 30 minutes or over a period of time required for gradual addition with constant stirring maintaining temperature to around 60°C for about 1 to 6 hours or for a period until the reaction is complete, thereafter allowing the reaction mixture to cool down to room temperature, recovering the product, washing the solids with aqueous methanol or another aqueous polar alcohol provides Sixth Composition in suspension that can be recovered, washed with 100 ml 90% methanol and air dried.

Suspending chitosan in alcohol preferably in 20 volumes, mixing well by stirring to achieve activation of chitosan, preferably at 100 rpm for 30 min, heating the said mixture to about 60°C, adding Phosphorus acid (H₃PO₃) dissolved in water and adding drop-wise to this mixture by stirring, adding formaldehyde about equal in weight to the dry weight of activated chitosan or its derivative in drop-wise manner maintaining the temperature between about 60 - 65°C, preferably at 63°C, for a period of time preferably to 2 - 6 hours after the addition is complete, allowing the suspension to cool provides the Seventh Composition in suspension that may be recovered. The said Seventh Composition may be soluble in the broad range of pH 7 to 10 giving slightly turbid to clear solutions,

Suspending chitosan in alcohol preferably in 20 volumes, mixing well by stirring to achieve activation of chitosan, preferably at 100 rpm for 30 min, heating the mixture to 60°C, adding formaldehyde 750 ml over a period of few minutes, adding and about 10% of inorganic acid or Phosphorus acid (H₃PO₃) dissolved in water and added drop-wise to this mixture by stirring for about one hour or over a period of time required for effective mixing, maintaining the temperature between 60 - 65°C and preferably at 63°C for about six hours or for a period of time required for completion of the reaction, allowing the suspension to cool after the addition is complete and provides Eighth Composition in suspension that may be recovered.

Suspending chitosan in alcohol preferably in 20 volumes, mixing well by stirring to achieve activation of chitosan, preferably at 100 rpm for 30 min, heating the mixture to 60°C, adding formaldehyde over a period of few minutes, adding about 10% inorganic acid or Phosphorus acid (H₃PO₃) dissolved in water and added drop-wise to this mixture by stirring for about 1 hour or over a period of time required for effective mixing, maintaining the temperature between 60 - 65°C and preferably at 63°C for 6 hours or for a period of time required for completion of reaction after the addition is complete, allowing the suspension to cool, recovering the suspension as dry powder, resuspending in 20 volumes of 90% methanol, and 2 volumes of concentrated HCl or any other organic or inorganic acid, stirring the mixture at room temperature for one hour or for a period of time required for good mixing, filtering out, washing with 20 vol 90% methanol provides Ninth Composition that may be recovered.

Mixing Fifth Composition in solution with a charged molecule in solution and coprecipitating the same, preferably in alkaline pH, provides Tenth Composition of this invention. Thus, adding chitosan to alcohol, adding water about 20% of volume of alcohol to prepare a mixture, heating and maintaining the said mixture to about 70°C, adding succinic anhydride to this mixture, stirring for one hour or for a period of time required for good mixing, maintaining the temperature between 60 - 70°C and preferably at around 65°C for 1 hour or for a period of time required for the reaction, allowing the suspension to cool to get a succinic derivative, adding Lactic or another acid about 10% of the dry weight of the succinic derivative, and per 5 gram dry weight of succinic derivative used adding enzyme for deaceylation about 4000 I.U, stirring the mixture for a period of time, preferably for 7 hours, adding further chitinolytic enzyme 500 I.U. per 5 gram dry weight of succinic derivative used and continuing stirring for a period of time, preferably for 1 hour, allowing the suspension to cool to get solid Fifth Composition, recovering and air drying the Fifth Composition, dissolving the Fifth Composition to make a 1% solution in water, adding an aqueous solution of copper sulfate or another charged molecule, capable of adsorbing on the Fifth Composition in alkaline pH, as a solution, adding the said solution of copper sulfate or the charged molecule that may be 1% with respect to water in a small quantity, about 1% of the volume of water taken for dissolving Fifth Composition and mixing for few minutes, adding liquid ammonia to precipitate chitosan with the bound copper sulfate or another charged particle provides a derivative of Tenth Composition.

This invention also comprises a composition comprising at least one of the compositions from Second and Fourth to Tenth Composition and derivatives thereof being used as an essential ingredient. The compositions may be made in dilute organic acid, usually acetic acid or in water. The said composition may be a composition for preservation of on fruits, vegetable, ingestible foods and feeds, delayed ripening, delayed ageing and delayed senescence of natural products including fruits, vegetable and flowers or functioning as a carrier for delivery of an active molecule or a functional group to a site of its action. The said composition may have effective concentration around 0.1 %, and the said acetic acid may be around 2% when used.

The chitinolytic enzyme used in this invention may be a proteolytic as well as chitinolytic enzyme. The illustrated embodiment of this enzyme has a molecular weight of 50 Kilo Daltons on SDS-PAGE electrophoresis, an optimum chitinolytic activity at pH 2 and an optimum chitinolytic activity at temperature of 80°C. It is an embodiment of this invention that *Aspergillus niger* has been identified as a source of this type of enzyme, although other sources may also be present and are included within the scope of this invention. *Aspergillus niger* MTCC 5572 has been identified as the organism capable of producing this enzyme in a good quantity. It is also possible that mutants derived from this strain may also possess capability to produce the proteo-chitinase useful to produce products of this invention. The enzyme was precipitated from crude culture filtrate by saturating the same with ammonium sulfate and fractionating the same on Sephadex^{®}. recovering crude proteo-chitinase enzyme by ammonium sulfate precipitation.

This invention also comprises a process of improving shelf life of fruits, meat, fish, flowers, vegetables comprising a treatment of dipping, spraying, fogging or another process of external as well as adding into the food product application of a composition comprising one or more of Second and fourth Composition to Tenth Composition. The said process may also be a process for slowing down ripening and/or microbial spoilage that may be applied to natural products that may include, without limitation, banana, mango, apple, strawberry, orange, lime and the like, and applications may include external application by coating or an "internal application" to cut or processes fruits or cut of processed natural products..

The deaceylating enzyme, the xylan acyl transferase, of this invention was isolated form *Bacillus sp* MTCC 5571. However, any other deaceylating enzyme from another microbes may give same results.

### DETAILED DESCRIPTION OF THE INVENTION

This invention embodies novel compositions of chitosan for treatment as anti-respiratory, anti-microbial and anti-oxidative for keeping freshness of natural products, processes for production of the said novel compositions, novel methods / processes for the said treatment to the said natural products for enhancing or improving their shelf life or storage stability. This invention further embodies a novel thermostable proteolytic microbial enzyme that has chitinase action even in presence of organic solvent and its use in developing novel chitosan compositions of this invention. This invention also embodies use of enzyme for de-acetylation of the chitosan.

The invention also embodies methods / processes of keeping freshness of natural products for longer time. The invention further embodies methods / processes of application of the said compositions and equipment useful for the purpose of applying the said compositions to the natural products, without limitation, comprising fruits, vegetables, meat, fish and other food products. Illustrative list of natural products covered by the scope of this invention includes food products and non-food natural living or non-living products, further including without limitation, fruits, vegetables, meat, fish, cut flowers, milk and food products in general.

Chitosan being polydisperse molecules with widely varying possibilities of deacylaiton, substitutions, sites of hydrolysis, composition of the hydrolysate etc. their properties that govern antimicrobial, anti-ripening, preservative properties, solubility, capability as carrier molecule etc. will differ with the final composition of treated chitosan. Efficacy and extent of change in chitosan molecule brought about by a given treatment will largely depend also on the extent to which the sites vulnerable for a chemical or physical change (adsorption) become available, which will differ with what treatment is given for "activation" of the chitosan molecule before any treatment for their derivatization is initiated. Since effect of any substituting or modifying treatment will depend on the functional group composition of the raw material chitosan, for example enzymatic hydrolysis of untreated and activated chitosan shall yield a different composition of enzymatic hydrolysate than the one where the chitosan molecule is already modified by a substitution and the extent to which it is modified by substitution, since enzymes are vulnerable to changes in the environment of their active sites. Properties of a substituted chitosan with respect to antimicrobial, preservative, physiological process affecting properties etc. are dependent on the treatment given to it, distribution of molecular weight of the product, the combination of functional groups it carries and degree of overall substitution, which opens up innumerable possibilities of the properties of the products and products themselves, which can seldom be predicted unless experimentally found out. Task of achieving the objective of improvement by routine experimentation is a daunting one since the permutations and combinations required to be worked out are infinite, and is perhaps a reason due to which the full scope of this explanation has not been covered so far.

Whereas it is customary to activate chitosan by giving a treatment of an acid to it, it was surprisingly found in this invention that chitosan gets activated for the purpose of preparing a derivative by simple heating of its suspension in alcohol that contains water from 0% to maximum of about 30%, water as per requirement and stirring for a period of time to get activated chitosan and thereafter reacting the said activated chitosan with a reagent capable of complexing by physical forces or chemical forces to prepare a derivative. A composition of chitosan prepared in this way, by further treatment and derivatization offers a series of carriers which can bind by physical or chemical bonds with functional groups / active molecules for improving their efficacy.

Chitosan derivatives when completely substituted, do not show antimicrobial activity. Large molecular weight chitosan has poor solubility. Unsubstituted low molecular weight chitosan shows both antimicrobial property and solubility. Therefore it is generally believed that low molecular weight chitosan is more useful as a preservative. However, low molecular weight chitosan compositions do not have good coating property when applied to fresh fruits and similar objects for a preservative action. It is a surprising finding of this invention that even high molecular weight chitosan, when derivatized as disclosed in this invention is effective preservative and in addition to anti-microbial property it also protects fruits from ethylene, and water loss due to its better coating property. Without being bound to the theory, it has been a current interpretation of this invention that the desired antimicrobial, preservative and physiological process affecting properties are optimal only in a certain range of molecular weight distribution as combined further with solubility profile and functional groups or active molecules carried by the chitosan derivatives. The methods / processes of production of chitin derivative of this invention are directed to achieve this narrow and intricate balance.

Throughout this specification, if it has not been separately mentioned, unless the context does not permit, in any context of a treatment of derivatization, chitosan is a product that is 50 to 70% deaceylated and of 60 - 100 mesh, although other degrees of deaceylation and/or mesh size may also be used within reasonable limits to get same results. Chitosan is always activated, before giving the said treatment, by suspending the said chitosan in absolute alcohol or with added water which may be up to about 30 % or less, stirring for a period of time to get activated chitosan and there after reacting the said activated chitosan with a reagent capable of forming a derivative by complexing by physical forces or chemical forces to prepare a derivative. Since, the method / process of activation of this invention has never been used in prior art, it opens up an entirely new platform for any derivative of chitosan including acid derivative, succinyl derivative as well as enzymatic hydrolyzates derived from chitosan activated by method / process of this invention.

Wherever "alcohol" has been mentioned in this specification, unless the context does not permit, preferred alcohol is a polar alcohol in which the Chitosan or their derivatives as prepared in course of process of this invention are insoluble. Most preferred alcohol is methanol, although within above limitations, other alcohols can also be used.

Wherever lactic acid is used, although it is the most preferred acid to be used for those reactions, any other acid, inorganic as well as organic can be used, although organic acid is more preferred and lactic acid is most preferred.

Solubility of the compositions in this specification was checked by adding 100 mg powder slowly to 100 ml distilled water (pH 6.5) over 5 min. The mixture was stirred continuously during addition and further for 10 more minutes and then the solubility was checked visibly. In one of the embodiment, that resulted in the First Composition of this invention, chitosan is dissolved in acid and the resultant solution shows anti-microbial activity. The quantity of the acid used in relation with chitosan as well as choice of acid are crucial in exhibiting the activity. The acid useful for this purpose may be any organic acid, although lactic acid is preferable. Similarly, in place of lactic acid, less preferred option could be any inorganic acid. Without getting bound to the understanding, particularly good performance of lactic acid for these processes could perhaps be due to limited solubility of chitosan in lactic acid at less than one part of lactic acid per part of chitosan that gives a fluid that has some viscosity and is not fully watery solution, giving the derivative an optimum mix of charge on the particle and functional group so that enough solubility is achieved without losing its antimicrobial properties. It was surprisingly found that under the reaction conditions of this invention, wherein chitosan is dissolved in acid at about 10% by weight of chitosan there was no solubility of the acid derivative (the First Composition) in water, and when proportion of the lactic acid was increased, the First Composition was soluble in water, but there was a lot of lump formation which made the product difficult to use and also did not have antimicrobial activity. It was a further surprising finding that, on the contrary, using a proportion of lactic acid lesser than 10% of weight of chitosan resulted into water soluble product that had antimicrobial activity. Since lactic acid below 0.5 ml gave complete dissolution without formation of lumps during the reaction, they were the preferred embodiments for using for the further work. Similar principles would work for other acids, and the actual quantity of the acid used will depend on the properties of acid.

In another embodiment of this invention, that embodies the Second Composition, activated chitosan was treated with Succinic anhydride to get a Succinic derivative. Succinic anhydride is added in different proportions by maintaining temperature at about 60 - 70°C, preferably at 65°C. When water alcohol mixture used for activation was in proportion of 20:80, the resulting Succinic derivative was soluble in water only when more than 0.5 gm Succinic anhydride was added to 5 gm of chitosan in the said reaction. It was however , surprisingly found that by changing the ratio of solvents used to 10 ml water to 90 ml methanol, it was possible to get water soluble product by using succinyl anhydride at a quantity lower than 0.5 gm up to 0.05 gm. For practical purposes, it is found that quantity of 0.1 gm was preferable. This observation further supports the finding that activation treatment is very critical to determine properties of the products and the novel activation method / process of this invention shall lead to all the products that shall have different properties from the prior art products of same reagent if the activation of chitosan was by a substantially different method / process.

Further embodiment of this invention comprises the Third Composition wherein in first step, the First Composition is prepared using an acid, preferably lactic acid at a rate of 10 to 20% of the dry weight of activated chitosan and the said First Composition is taken as a starting material and reacted with succinic anhydride added at a rate of about 10% of the dry weight of the First Composition taken. This composition is a coarse granular material that was soluble in water.

Still further embodiment of this invention comprises the Fourth Composition wherein a succinic derivative of activated chitosan of the Second Composition is prepared using succinic anhydride at about 10% of the dry weight of activated chitosan and after the reaction is over, an acid, preferably lactic acid at 10 to 20% of the dry weight of Chitosan was added. There was no lump formation. The dry powder was completely soluble in water within 5 min under the described test conditions.

In a further embodiment of this invention a class of electrophoretically homogeneous enzymes isolated from microorganisms has been found to have a proteolytic activity as well as chitinase activity, which may be designated as proteo-chitinases. In an embodiment of this invention, the said proteo-chitinase and enzymes capable of deacetylation of chitosan are used to hydrolyse chitosan, in a process to make products of this invention. Although proteo-chitinases and deacetylases of this invention are isolated from microbes, this class of enzyme, derived from whichever source shall perform the same function of hydrolysis of chitosan and this invention extends to their use also for the purpose of chitosan hydroysis and further for developing products of this invention.

Soil samples were collected, in and around Pune, India, from various natural resources rich in organic matter, enriched with the desired substrate including casein, chitosan, washed wheat bran, cellulose. The enrichment was continued for 3 weeks, and the samples were streaked on solid media containing the substrate used for enrichment as sole source of carbon. The isolated colonies generally showed zone of clearance indicating the strong production of the respective degradative enzyme.

About 700 cultures were isolated for various bacteria and fungi. To find out cultures having desired activity, they were cultured by shake flask method. Crude microbial culture filtrate was concentrated by ammonium sulfate precipitation. The protein fractions were separated by denaturing SDS PAGE electrophoresis. The protein fractions were separated on these gels as homogeneous independent bands. One culture of *Aspergillus niger* MTCC 5572 was selected as promising culture.

The electrophoretically homogeneous band isolated from culture filtrate of *Aspegillus niger* MTCC (deposited at The Microbial Type Culture Collection (MTCC), Institute of Microbial Technology, Chandigarh, India) that showed proteinase as well as chitinase activity - a Proteo-chitinase, enzyme is novel one with a molecular weight of 50 KD as determined by molecular weight markers on SDS PAGE, and optimum enzyme activity at pH 2 and 80°C. Although this band was used for hydrolyzing enzymatically deacylated Second Composition of chitosan for developing product of this invention, any other proteo-chitinase may give hydrolysate with closely similar properties.

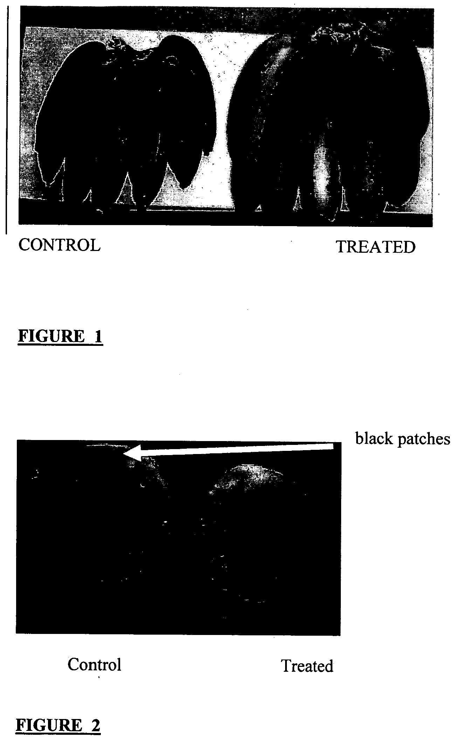

In a further embodiment of this invention chitosan hydrolyzate are obtained by using purified enzymes or crude form of enzymes or active form of microbial cell extract. The said hydrolysate shows improved anti-microbial activity upto a certain limit of decrease in average molecular weight, and within specific range of degree of substitution of the free amino group by either acetyl or any other anionic group. In a further embodiment of the invention, enzymatically hydrolyzed derivatives of chitosan show better anti-microbial activity than only chemically derived product. The hydrolysate modified further, i.e. de-acelyated using xylan acyl transferse shows further improvement in anti-microbial, anti-oxidant property and acts as an anti-respiration agent too. The chemically derived hydrolysates having molecular weight in lower range of 8 - 100 kD are more effective than higher and / or lower molecular weight hydrolysates. However, surprisingly, after enzymatic deacetylation, even high molecular weight derivatives show antimicrobial activity equivalent to that of 8 -100 KD products derived by chemical process. Preferred method / process of getting xylan acyl transferses in this invention is from microorganisms. These enzymes, isolated as crude or purified extract from any other source can be predicted to have the same deacylating properties and shall yield the same products as are prepared in the preferred embodiments of this invention from microorganisms. This embodiment of invention comprising enzymatic hydrolysates comprises Fifth Composition that is useful as a post harvest preservative. The said Fifth Composition was prepared by first preparing a succinic derivative of Second Composition to which Succinic anhydride is added, the derivative is deacylated by enzyme in presence of an acid, preferably a lactic acid, added at about 10% by weight of the dry weight of the succinylated. Second Composition for a period of time to achieve limited deaceylation, preferably for a period of 7 hours, and then subjected to about one hour treatment of chitinolytic enzyme. The powder of Fifth Composition is soluble in water when more than 0.5 gm succinic anhydride is added to 5 gm of chitosan in the said reaction and displayed antimicrobial activity. Fifth composition showed three to four times less or lesser consumption per kg of banana and tomato than the solution of chitosan in acetic acid, showed higher antimicrobial as well as microbiostatic activity as compared to chitosan solution in acetic acid and First, Second and Fourth Composition. Fifth Composition was also useful in improvement of shelf life of fruits. Freshly harvested (<30 h of harvesting) green banana when treated using 0.1% solution of the Fifth Composition. The treatment can also be given by dipping of banana in the said solution for nominally 10 - 15 sec., or by direct spray of the solution on the banana with a suitable device so as to make the surface of banana wet, or by passing the banana through a closed chamber which is saturated with the fog of the said solution (indirect fogging). The treated banana are air dried. These banana are stored at room temperature (20 - 32°C) and a relative humidity of 40 - 60%. In experiments, the treated banana maintained green color, and weight where as the untreated banana lost weight and developed discoloration. The treated banana showed better physiological condition as tested chemically by determination of their total solids (BRIX contents), reducing sugars, and weight loss at least up to 12 days after dipping (see Fig 1). It is a further embodiment of the invention that further to the storage for 8 - 15 days, when ripened by application of ethylene, the treated banana ripened normally, whereas the untreated control banana either became hard and did not ripen, or turned black and soft and did not remain fit for human consumption.

The whole tomato and cut apple were treated with the product from example 10, exactly as described in example 14 for banana. The treated tomato retained its weight, texture and freshness for 30 days as against untreated control tomato, which wrinkled in 12 - 15 days resulting in weight loss. Physiological changes in the treated and control tomatoes were monitored on 7^{th} day of storage using MRI scanning technique. The fleshy skin of the treated tomato was more uniform in its color density. The fleshy skin of the untreated control tomato has dark patches that are indicative of uneven and more drying. The MRI scan of the representative tomatoes is included in the Figure 2.

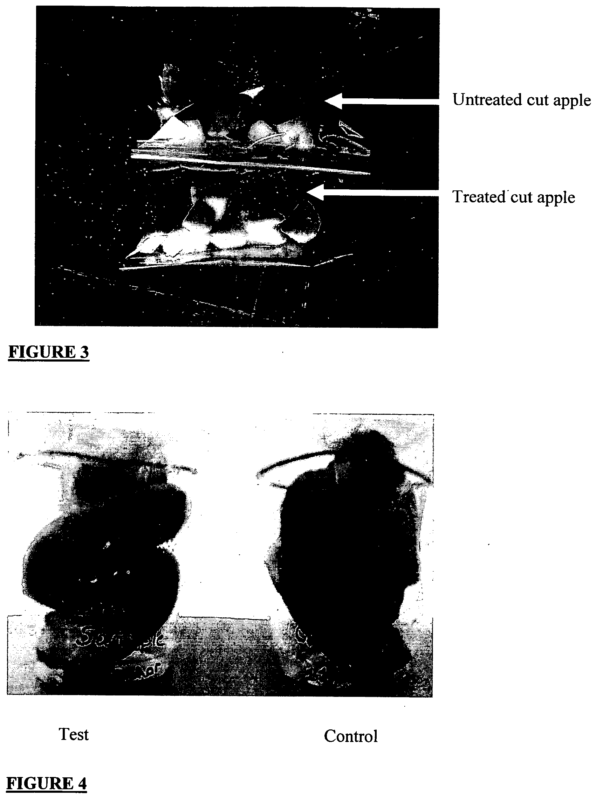

Fifth Composition is effective also for "internal application: i.e. for application to preservation and for improvement of storage life or for antimicrobial treatment or microbiostatic treatment to "internal" parts of fruits or natural products such as cut fruits, processed fruits, processed vegetable products and the like. The pieces of cut apple were packed in polythene bag or transparent plastic container and stored in refrigerator at temperature of 4 - 9°C. The cut apple from control started becoming brown from day 2 and turned soft by Day 3, whereas the treated cut apple retained its color, taste, and hard texture for 6 days. The photograph (Figure 3) shows the difference in the appearance of treated and untreated cut apple on day 4.

When treated and untreated cut apple pieces were packed in transparent plastic container and stored as described above for up to 25 days. The untreated cut apple developed fungal growth [black patches in the photograph in Figure 4], whereas the treated cut apple did not develop any fungal or other microbial growth. The results are depicted in the following photograph.

In a further embodiment of this invention, the Fifth Composition serves as a carrier for functional groups or molecules for improvement in efficacy at lower concentrations and/or as carriers for their delivery to active site. Illustrations of this embodiment are provided by Sixth and Tenth Compositions.

Keeping the principle same, several more embodiments are possible for Fifth Composition as a carrier by varying the functional groups or active molecules that are carried by the Fifth Composition by physical bonding or chemical bonding.

Sixth Composition comprises a chitosan succinate sugar derivative that shows variable solubility in water and also shows varying anti-microbial property. Sugar may be cane sugar or any other sugar. Method / process of this invention for preparing Sixth Composition comprises making a sugar derivative of Fifth Composition, which was further reacted with formaldehyde for a varying period of time, usually from one to six hours, to get the said Sixth Composition that had excellent water solubility and anti-microbial activity that is better than Fifth Composition when sugar solution used for derivatization was 0.4 g for each 100 g of dry weight of Chitosan succinate or less. It was a surprising observation that if sugar is used more than this level, the chitosan-succinate-sugar derivative was no more water soluble. This is a further support to the understanding that the derivatives of chitosan to be useful for applications should have a delicate control on degree of substitution and the functional groups to be used for substitution. All water soluble products of the Sixth Composition had varying degree of anti-microbial activity at various concentrations, which was consistently better than that of anti-microbial activity of the starting material Fifth Composition. When sugar used in above method / process was glucose or lactose taken at the rate of 0.4 gm per 100 gm chitosan succinate and period of reaction with formaldehyde was about 3 hours, the products of these reactions were soluble in water and showed 100% anti-microbial activity against E coli by the method / process described in Example 11.

In another embodiment of this invention chitosan includes derivatives prepared from an inorganic acid. This is exemplified by Seventh Composition where phosphorus acid is used to give a derivative and comprises suspending chitosan in alcohol (about 99%), mixing the suspension well by stirring at 100 rpm for 30 minutes, heating the mixture and maintaining the temperature to about 60°C, adding Phosphorus acid (H₃PO₃) dissolved in water of a volume that is about four times the weight of chitosan drop-wise to this mixture accompanied by stirring for about1 hour, further adding Formaldehyde 5 ml in drop-wise manner and after the addition is complete, maintaining the temperature between 60 - 65°C and preferably at 63°C for 2 - 6 hours. In place of formaldehyde, any other aldehyde can be used. The conditions of temperature, stirring etc. may be varied to get a final product with desired level of antimicrobial activity. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The solubility is also checked at pH 8, 9, and 10 adjusted using dilute NaOH, or Tris buffer. The products formed using chitosan and phosphorus acid in 1:1 and 1:2 ratio showed solubility in the broad range of pH (7 - 10), forming slightly turbid to clear solutions. Whereas antimicrobial compositions of chitosan or their derivatives in the prior art are with pH in the acidic side and the precipitate near pH 7 or in alkaline range, the Seventh Composition is soluble at pH 7 to 10 on alkaline side. These solutions showed varying degree of anti-microbial activity. Products up to 0.01% showed some antimicrobial activity whereas, products above this concentration showed total microbial inhibition. It is clear that the Seventh Composition, being soluble as well as of total microbiocidal activity at reasonably low concentrations and at pH 7 and above up to 10 provides new scope for their use as preservatives and antimicrobials.

A further embodiment of this invention comprises Eighth and Ninth Composition. Method / process of its preparation comprises reacting chitosan suspended in minimum 99% alcohol, mixing the suspension well by stirring for a period of time enough for getting efficient wetting and activation with alcohol, preferably at 100 rpm for 30 minutes, heating and maintaining the temperature of the mixture to around 60°C, adding Formaldehyde at the rate of about 5% of the volume of alcohol taken, adding drop-wise a 10% solution of Phosphorus acid (H₃PO₃) in water, the weight of Phosphoric acid being about 5% of the volume of alcohol taken and stirring further for the period of time, preferably for 1 hour. The temperature is maintained between 60 - 65°C and preferably at 63°C for 6 hours after the addition is complete. The suspension is allowed to cool and filtered, the solids recovered and dried. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The solubility is also checked at pH 8, 9, and 10 adjusted using dilute NaOH, or Tris buffer. The product dissolved in water, but precipitated out when the pH was raised to 7.0.

Ninth Composition was obtained by subjecting the product of Eighth Composition to HCl treatment. Although HCl has been used here, it may be replaced by any other organic or inorganic acid The dry powder obtained in this reaction was resuspended in 20 volumes of 90% methanol, and 2 volumes of concentrated HCl to get Ninth Composition in suspension in the reaction mixture. The mixture was stirred for one hour at room temperature. The powder was filtered out, washed with 20 vol 90% methanol 3 times and dried as described above. The resultant product was soluble from pH 3 to 12. This solubility over a broad range further expands the scope of application of this product. It is pertinent to note here that Phosphoric acid treatment is critical for priming the product for the solubility over a broad range of pH. This has been shown by the fact that other compositions, particularly Fifth Composition and Seventh Composition when treated with HCl as described in this example did not alter their profile of pH of solubility to the range of pH 3 to 12.

The Tenth Composition is one more illustrative embodiment of Fifth Composition acting as a carrier for an active molecule. In this instance, the active molecule is copper sulfate and presumably the mechanism of complex formation is by adsorption. The method / process of preparing the Tenth Composition comprises making 1% solution of Fifth Composition in water, adding about 10% of its volume of a (1%) solution of copper sulfate, mixing the two solutions for some time, adding liquid ammonia at the rate of one part to hundred parts of water taken, resulting into precipitation of chitosan with the bound copper sulfate. Copper sulfate is freely soluble in liquid ammonia and the resulting solution is deep blue in color. Estimation of copper is done by colorimetry, and using a standard graph 0.02 - 0.1%. From this the chitosan bound copper was estimated to be 90%. This is an example of possible use of the derivative as a carrier for all charged molecules.

Without getting bound to any theory, it is our understanding that chitosan has positive charge, and any acid whether organic or inorganic will form co-ordinate bond with the charged amino group on chitosan. These complexes have been named as respective salts of chitosan e.g. chitosan hydrochloride, chitosan acetate etc. These salts dissolve in water, and the pH of the solution is generally acidic. The salts also do not posses anti-microbial activity. When we try to increase the pH of these solutions by addition of any alkali - typically NaOH, the chitosan precipitates before the pH has become neutral. This is because the salt is not stable at the said pH. In our processes we have two types of modifications which are unique:
(1) Reaction of chitosan carried out in alcohol, with 0 - 30% of water providing limited hydration and therefore important in achieving controlled/ limited substitution of the side groups.
(2) Formaldehyde mediated reaction and anhydride mediated reaction. Both these reaction products are generally stable at akaline pH. Now the solubility of the product at alkaline pH is a function of net charges on the molecule. These can be added within either of the said 2 reactions, or by simple ionic interaction (salt formation) with any acid. Typically inorganic acids are preferred as the salts are more stable.
(3) The soluble chitosan derivatives developed by various methods / processes described herein have partial substitution and retain both positive and negative charges. Thus different charged molecules are attached to chitosan molecule, thereby rendering it useful as a carrier molecule for active groups, such as antimicrobial groups, or any other active group that have some functional purpose. The result is that the efficacy of a functional group that is attached to such molecule improves at a very small concentration. Herein, water soluble chitosan compositions prepared by the methods / processes of invention have been seen to be effective carriers of n acetyl cysteine and Copper Sulfate residues, the later has been demonstrated in Example 19. Same performance is expected to occur for any other functional group whose residue can be as effective as when it is in an unbound state when not carried by water soluble chitosan molecule. Water soluble chitosan molecule used here is prepared by methods / processes of this invention. However, water soluble chitosan molecule prepared by any other existing or inventive method / process shall be equivalent to this molecule for the purpose of its application as a carrier molecule and shall be considered as being covered within the scope of this invention unless there is any reason for which it can not carry any other active for any reason.

A yet another embodiment of this invention discloses a method / process of enhancing storage stability of natural products by treating with chitin derivatives. One embodiment of this aspect of invention discloses a method / process of enhancing storage stability of natural products by treating with chitin derivatives that retard life activities that are responsible for reducing storage stability or lead to ageing by slowing down or arresting such activities. Illustrative list of the said life activities include, without limitation, respiration, oxidation, ethylene formation, transpiration, as well as arresting aging of the fresh fruits, vegetables, meat, fish, processed and unprocessed food preparations and like. A person of ordinary skill in the art will be able to extend this list to many other life activities that reduce storage stability. For achieving above objective, the natural products may be coated with compositions of this invention by the methods which are well known to a person skilled in the art. The said coating can be full or partial and can be obtained by use of a method / process of application. The said method of application includes, without limitations, spraying, dipping, micro-spraying or by use of nano-particles, indirect fogging and like. In case of processed food such as bread, noodles etc. the said formulation can be added in the dough, and / or applied to the surface of the processed food by one of the various methods listed above.

When banana are stored for a long time the crown of the bunch starts drying. The stem which joins the banana finger to crown is called pedicel. It starts wilting. Wilting of pedicel is a best indicator of the drying. Less wilting means preservation of freshness.. The test was conducted on 1000 kg banana each by the standard method (control) dipping in 0.1 % of our product, spray of our product. This was repeated on 2 farms (total 6000 kg banana). Very low percentage of pedicel wilting by dipping as well as spraying of the test composition shows its good efficacy in delaying in drying and ultimately on delaying ageing and senescence and improving storage capability.

The results are given in the below Table 6. By dipping method significant improvement in the wilting (weight loss, or preservation of freshness) was observed.

In the following are described experiments conducted that serve as non limiting illustrations of how the invention is performed. Any modifications or variations in the parameters, including but not limited to process for producing hydrolysates and further modifications, the method / process of use of the products and their various steps used are merely illustrative and any equivalents of them that are obvious to a person skilled in the art and capable of achieving the same objective may be used in their place and yet they shall be considered as included in the scope of this specification.

Figures and their description:
Figure 1: Photograph of the untreated (left) and treated (right) banana on 12^{th} day of the storage.
Figure 2: The MRI scan of the representative tomatoes.
Figure 3: A photograph of difference in the appearance of treated and untreated cut apple on day 4.
Figure 4: The untreated cut apple (control) developed fungal growth visible as black spots of fungal growth in the photograph whereas treated cut apple (Test) remained without fungal growth after 25 days of storage in polythene bag.
Figure 5: Purification of proteo-chitinase. Gel filtration on Sephadex G-100.
Figure 6: Molecular weight determination of the proteo-chitinase
Figure 7: Optimum pH of proteo-chitinase. X axis shows pH and Y axis shows enzyme activity (units/ml) of a unit volume of single enzyme formulation. The maximum activity of 160 Units/ml is observed at pH 2.
Figure 8: Optimum Temperature of proteo-chitinase. X axis shows temperature and Y axis shows enzyme activity (unit/ml) of unit quantity of single enzyme formulation. Maximum activity is obtained at Temperature of 80 °C.
Figure 9: Action of selected enzymes on chitosan. Lane 1: Glucosamine standard. Lane 2: Xylan Acyl Tranferase (crude), Lane 3: Protease and Xylan Acyl Transferase (both pure). Lane 4: Xylan Acyl Transferase (pure). Lane 5: Chitosan without enzyme. Acetate spots (Results of de acetylation) are marked by circle.

### EXAMPLE 1

### PRODUCTION OF WATER SOLUBLE AND INSOLUBLE CHITOSAN LACTATE

Chitosan [70 DDA (Degree of Deacylation), 100 mesh] 5 gm is taken in a round bottom flask and 20 ml distilled water and 80 ml methanol (99%) is added to it. Any other grade of chitosan can be used here. The suspension is mixed well by stirring at 100 rpm for 30 min. An acid, inorganic or organic, preferably Lactic acid is added in different quantities to this mixture and stirring is continued for 2 hours. The temperature is maintained below 30°C and preferably at 25°C. The suspension is filtered and the solid is air dried. The free flowing powder and lumps (if any) are separated by sieving. The free flowing powder is then further dried in hot air oven [70°C for 3 - 6 hours]. Lumps are elastic particles that do not break to free flowing particles of 100 mesh or smaller. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The effects of different concentrations of lactic acid are given in the Table 1.

**Table 1: Effect of different quantities of lactic acid used with 5 gm chitosan.**

| Lactic acid (ml) | Formation of lumps during the reaction | Solubility in water |
|---|---|---|
| 6.5 | +++ | Dissolves completely |
| 5.0 | ++ | Dissolves completely |
| 3.5 | + | Dissolves completely |
| 2.0 | - | Dissolves completely |
| 1.0 | - | Forms lumps that dissolve after 30 min |
| 0.5 | - | Forms lumps that do not dissolve up to 60 min. |
| 0.1 | - | Dissolves completely when reaction is carried out in 90:10 methanol: water |

| | | |
|---|---|---|
| +++ indicates 60% or more lump formation. ++ 35% lump formation + about 15% lump formation and lump size is small _no lump formation | | |

Since lactic acid below 0.5 ml gave complete dissolution without formation of lumps during the reaction, they were the preferred embodiments for using for the further work.

### EXAMPLE 2

### ADDITION OF SUCCINATE GROUP TO CHITOSAN

Chitosan [70 DDA, 60 mesh] 5 gm is taken in a round bottom flask and 20 ml distilled water and 80 ml methanol (99%) is added to it. Any other grade of chitosan can be used here. The suspension is mixed well by stirring at 100 rpm for 30 min. The mixture is heated to 65°C. Succinic anhydride is added in different proportions ranging from 15 g to 0.1 gm to this mixture and stirring is continued for 1 hour. The temperature is maintained between 60 - 70°C and preferably at 65°C for about 1 hr.

The suspension is allowed to cool and filtered and the solid is air dried. The free flowing powder is formed by breaking the loose lumps mechanically and sieved using 100 mesh. No lumping was observed. The free flowing powder is then further dried in hot air oven at 85°C for 3 - 6 hours. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The powder is soluble in water when 0.5 gm or more succinic anhydride is added to 5 gm of chitosan in the said reaction.

### EXAMPLE 3

### TWO STEP DERIVATIZATION WITH AN ACID AND SUCCINIC ANHYDRIDE

The reaction is carried out exactly as described in example 2 wherein the chitosan is replaced by the reaction product obtained in a reaction described in example 1 where 1.0 gm or 0.5 gm lactic acid is used. In place of lactic acid, any other inorganic or organic acid could have been used. Further 0.5 gm of succinic anhydride was used for 5 gm of the product of the First Composition. Coarse granular material was formed which was soluble in water when tested as described in example 1.

### EXAMPLE 4

### SEQUENTIAL ADDITION OF SUCCINATE AND LACTATE TO CHITOSAN

The reaction was carried out as per example 2 wherein 5 gm chitosan is reacted with 0.5 gm succinic anhydride. After the reaction was complete and the reaction mixture was cooled to 30°C and lactic acid - 0.5 or 1.0 ml was added. Lactic acid could have been replaced by any other inorganic or organic acid. The mixture was stirred for 1 hour, filtered and processed further as described in example 1. There was no lump formation. The dry powder was completely soluble in water within 5 min under the described test conditions.

### EXAMPLE 5

### IDENTIFICATION OF ENZYME HAVING PRIMARY PROTEINASE ACTIVITY AND CHITINASE CROSS REACTIVITY

Soil samples were collected, in and around Pune, India, from various natural resources rich in organic matter. The soil sample 10 gm was enriched with the desired substrate (1 gm) namely casein, chitosan, washed wheat bran, cellulose individually in separate 250 ml flask, containing 100 ml sterile distilled water. The enrichment was continued for 3 weeks, and the samples were streaked on solid media containing the substrate used for enrichment as sole source of carbon. The isolated colonies generally showed zone of clearance indicating the strong production of the respective degradative enzyme. About 700 cultures were isolated for various bacteria and fungi.

To find out cultures having desired activity, they were cultured in 1 liter flasks - containing 200 ml medium by shake flask method. Crude microbial culture filtrate was concentrated by ammonium sulphate precipitation dissolved in 0.1% SDS. The protein fractions were separated by denaturing SDS PAGE electrophoresis. The protein fractions were separated on these gels as homogeneous independent bands.

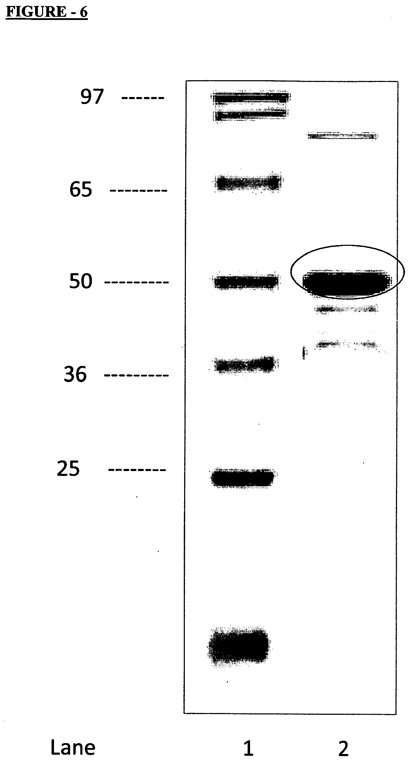

These bands were characterized for their activity by three different methods / processes; coumassie blue staining for protein detection, casein clearance assay for proteinase activity and Ramezol Brilliant Blue Chitin clearance assay for chitinase activity. An electrophoretically homogeneous band was detected that showed proteinase as well as chitinase activity - Proteo-chitinase. This band was used for developing hydrolysate of chitin for developing product of this invention. The molecular weight of the purified proeto-chitinase of selected *Aspergillus sp.* was determined to be 50 KD using molecular weight standard on SDS PAGE. The tracing of the gel electrophoresis pattern is shown in the figure 6. Based on the assay, *Aspergillus niger* MTCC 5572 (deposited at The Microbial Type Culture Collection (MTCC), Institute of Microbial Technology, Chandigarh, India) was selected for its proteo-chitinase activity and *Bacillus sp.* MTCC 5571 (deposited at The Microbial Type Culture Collection (MTCC), Institute of Microbial Technology, Chandigarh, India) was selected for its deacetylation activity and were taken for production and isolation of enzyme on large scale.

### EXAMPLE 6

### PRODUCITON OF PROTEASE CAPABLE OF CLEAVING CHITIN AND CHITOSAN

Microbial culture(s) were identified that are capable of production of acid or neutral protease(s) and endo-chitinase. The enzyme fraction isolated from the respective microbial cultures that had proteinase as well as chitinase activity (proteo-chitinase) was incubated with 1 % chitosan solution in 1 % acetic acid. The protease degraded the chitosan partially resulting in reduction in the viscosity of the substrate. The cultures identified to posses promising proteinase as well as endo-chitinase reaction (Proteo-chitinases) were grown on a large scale for enzyme production. Specifically selected was *Aspergillus niger* MTCC 5572 which was grown under shake flask conditions in casein yeast extract medium. The culture filtrate was characterized for enzyme activity, and the crude proteo-chitinase enzyme was recovered by ammonium sulfate precipitation [90% saturation]. The enzyme activity of the ammonium sulfate precipitate was determined by protein determination by colorimetric method, and known quantity of proteo-chitinase enzyme was added to chitosan for its modification. The crude enzyme from *Aspergillus niger* MTCC 5572 was concentrated by ammonium sulfate precipitation was dissolved in phosphate buffer pH 7.0. This was subjected to Gel filtration using Sephadex^{®} G-100. Different fractions were collected that were assayed for protein content and enzyme activity.

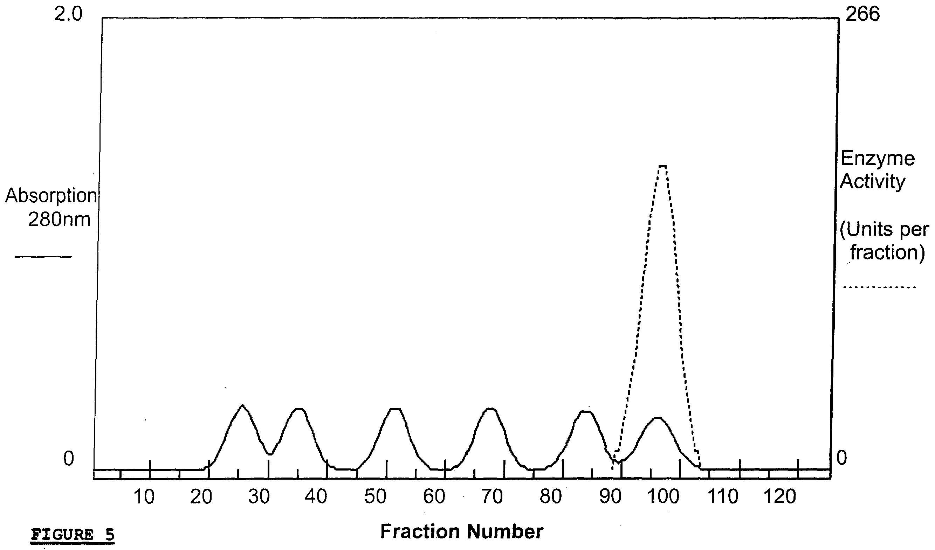

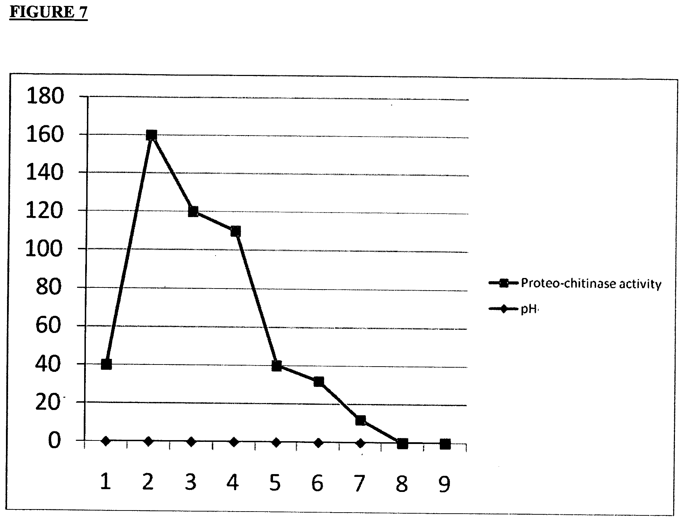

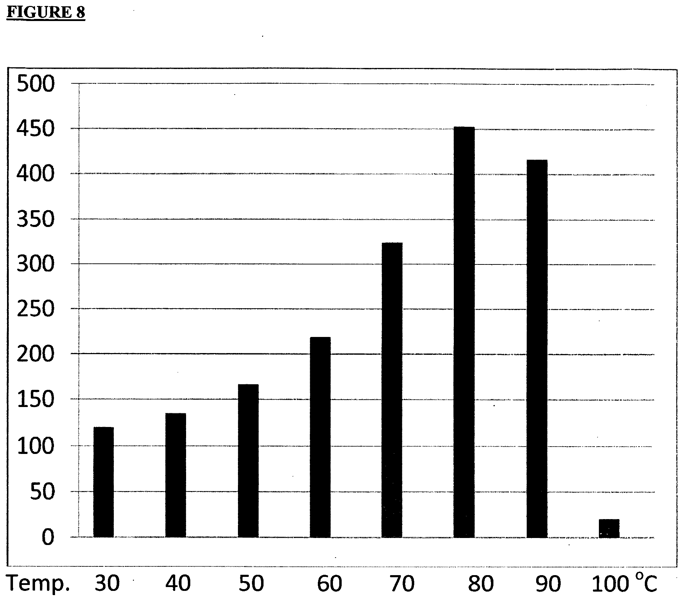

Fraction no. 89 to 104 corresponded with the single protein peak (see figure 5). This method / process was used for purification of the enzyme from 3 - 12 L broth in a single batch. The optimum pH and optimum temperature for the proteo-chitinase was determined over pH range of 1 - 9 and temperature range of 30 - 100°C The enzyme showed optimum activity at pH 2 (Figure 7), and 80°C (Figure 8).

Culture(s) of *Bacillus sp* were identified that are capable of production of Xylan acid transferase i.e. a deaceylating enzyme and was incubated with 1% chitosan solution in 1% acetic acid. The cultures identified to posses promising deaceylation activity were grown on a large scale for enzyme production. Specifically selected was *Bacillus sp* MTCC 5571 which was grown under shake flask conditions in casein yeast extract medium. The culture filtrate was characterized for enzyme activity, and the crude proteo-chitinase enzyme was recovered by ammonium sulfate precipitation [90% saturation]. The enzyme activity of the ammonium sulfate precipitate was determined by detection of acetate spot in TLC by treatment of chitin with the enzyme fraction, and known quantity of deaceylating enzyme was added to chitosan for its modification. The crude enzyme from *Bacillus sp* MTCC 5571 was concentrated by ammonium sulfate precipitation was dissolved in phosphate buffer pH 7.0. This was subjected to Gel filtration using Sephadex^{®} G-100. Different fractions were collected that were assayed for protein content and enzyme activity. This method / process was used for purification of the enzyme from 3 - 12 L broth in a single batch.

### EXAMPLE 7

### PRODUCTION OF XYLAN ACYL TRANSFERASE CAPABLE OF DEACETYLATING CHITIN AND CHYTOSAN

Microbial culture(s) were identified and isolated that are capable of production of xylan acyl transferase. The deacetylation of chitosan under the test conditions was done by these enzyme(s). The reaction mixture showed solubilization of chitin as viscosity of the solution increases. The occurrence of free acetyl group is detected by TLC. The cultures identified to possess chitin de-acetylation property were grown on large scale for enzyme production. Cultures selected was of *Bacillus sp.* MTCC 5571 and was grown under shake flask conditions in wheat bran yeast extract medium. The culture filtrate was characterized for enzyme activity, and the crude enzyme was recovered by ammonium sulfate precipitation [90% saturation]. The enzyme activity of the ammonium sulfate precipitate was determined, and known quantity of enzyme was added to chitosan for its modification.

### EXAMPLE 8

### ENZYMATIC HYDROLYSIS OF CHITOSAN

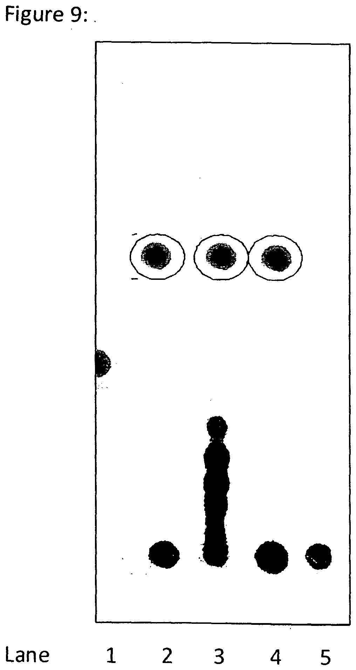

Chitosan 5 gm was added to methanol 70 ml and distilled water 30 ml. This mixture was stirred continuously at 100 rpm. Enzyme capable of cleaving chitin [described in example 5] was added at 10 - 1000 I.U. per gm chitosan concentration. The reaction was carried out at temperatures ranging from 25 - 95°C for 30 min to 5 hours. The preferred conditions were 100 I.U. enzyme per gm chitosan, temp. of 50°C and an incubation period of 2 hours. The reaction product was recovered by filtration and drying as described in example 2. The unmodified chitosan [1 gm] when dissolved in 100 ml distilled water acidified using 2 ml acetic acid solution, showed viscosity of 80 - 100 cps. The enzymatically modified chitosan [1 gm] when dissolved in 100 ml distilled water acidified using 2 ml acetic acid solution, showed viscosity of 20 - 30 cps. In order to prove that the said enzyme works on chitosan, purified enzyme (0.1% wt/vol) was added to 0.5% chitosan solution (made in aqueous 0.5% acetic acid), and incubated at 50°C for 16 h. The suspension was centrifuged, and the clear supernatant was analyzed by TLC (Solvent system n propanol:water: ammonia 7:2:1) using glucosamine as standard. The enzyme that showed proteo-chitinase activity produced chito-oligosaccharides which developed as spots on TLC which were slow moving as compared with glucosamine. The proteases from different organisms that did not show cross chitinase activity had no spots that moved on the TLC plate (figure 9).

### EXAMPLE 9

### ENZYMATIC DEACETYLATON OF CHITIN

Chitin 5 gm was added to 100 ml distilled water. This mixture was stirred continuously at 100 rpm. Enzyme capable of de-acetylation of chitin [example 6] was added at 500 - 1000 I.U. per gm chitin concentration. The reaction was carried out at temperatures ranging from 25 - 95°C for 8 to 24 hours. The preferred conditions were 800 I.U. enzyme per gm chitin, temp. of 50°C and an incubation period of 8 hours.

The reaction product was recovered by filtration and drying as described in example 2. The unmodified chitin was completely insoluble in water. The enzymatically modified chitin [10 gm] when stirred in 100 ml distilled water acidified using 2 ml acetic acid solution, showed viscosity of 30 - 50 cps, indicating partial solubilization of chitin as a result of de-acetylation.

### EXAMPLE 10

### THE POST HARVEST PRESERVATIVE CHITOSAN COMPOSITION AND PREFERRED PROCESS OF MANUFACTURING OF THE SAME

Chitosan [50 - 70 DDA, 60 mesh] 5 gm is taken in a round bottom flask and 20 ml distilled water and 80 ml methanol (99%) is added to it. Any other grade of chitosan can be used here. The suspension is mixed well by stirring at 100 rpm for 30 min. The mixture is heated to 65°C. Succinic anhydride is added in different proportions ranging from 15 g to 0.1 gm to this mixture and stirring is continued for 1 hour. The temperature is maintained between 60 - 70°C and preferably at 65°C The suspension is allowed to cool. Lactic acid 0.5 ml, and enzyme for deaceylation 4000 I.U. produced from *Bacillus sp* MTCC 5571 was added and the mixture was stirred for 7 hours. Lactic acid could have been replaced by any other inorganic or organic acid.

Chitinolytic enzyme 500 I.U. produced from *Aspergyllus niger* MTCCdescribed in example 5 was added and the stirring was continued for 1 hour. The suspension is allowed to cool and filtered and the solid is air dried. The free flowing powder is formed by breaking the loose lumps mechanically and sieved using 100 mesh. No lumping was observed. The free flowing powder is then further dried in hot air oven at 70°C for 3 - 6 hours. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The succinylated powder is soluble in water when more than 0.5 gm succinic anhydride is added to 5 gm of chitosan in the said reaction.

### EXAMPLE 11

### PRODUCTION OF WATER SOLUBLE AND INSOLUBLE CHITOSAN SUCCINATE - SUGAR DERIVATIVE CARRYING FOMALDEHYDE RESIDUES

5 gm of Chitosan succinate derivative as described in example 10, is taken in a round bottom flask and 100 ml methanol (99%) is added to it. The suspension is mixed well by stirring at 100 rpm for 30 min. and heated to 60°C. The temperature was maintained through out the reaction. Sucrose - in the range of 1 gm to 0.02 gm (see Table No. 3) was dissolved in 10 ml water, and same was added to the suspension.

Mixture was stirred for 15 min. Formaldehyde 5 ml was added to this mixture drop-wise over 30 min. The reaction was continued at 60°C with constant stirring further for 1 to 6 hours. This is termed as reaction time in the Table No. 3. The reaction mixture was allowed to cool down to room temperature. Subsequently the product was recovered by filtration. The solids were washed with 100 ml 90% methanol and air dried. The product was characterized as free flowing powder or lumps. The solubility of 0.5 % powder was determined in distilled water (pH 6.5) and 1% acetic acid. The results of these experiments are summarized below:

**Table. No. 3.**

| Sucrose in gm | Reaction time in Hours | Product form | Solubility in water | Solubility in 1% acetic acid |
|---|---|---|---|---|
| 1 | 6 | Powder | Insoluble | Insoluble |
| 0.5 | 6 | Powder | Insoluble | Insoluble |
| 0.1 | 6 | Lumps/Gel | Soluble | Insoluble |
| 0.02 | 6 | Lumps/Gel | Soluble | Insoluble |
| 0.02 | 3 | Lumps/Gel | Soluble | Soluble |
| 0.02 | 1 | Powder | Soluble | Soluble |

All the water soluble products had varying degree of anti-microbial activity, which was consistently better than that of anti-microbial activity of the starting material (product of example 10).

The anti-microbial activity of these products was tested using *E. coli,* and the protocol described in example 14. The results of the test are as follows:

**Table 4: initial count of E coli was 4X 10⁷**

| Product/ concentration | 1 % | 0.5% | 0.1% | 0.033% |
|---|---|---|---|---|
| Product of example 10 | 2X 10³ | 2X 10³ | 4X 10³ | 1X 10⁴ |
| Product 10 with sucrose 0.1 gm | 7X 10² | 4X 10² | 4X 10² | 1X 10³ |
| Product 10 with sucrose 0.02 gm 6h | 5X 10² | 2X 10² | 4X 10² | 90 |
| Product 10 with sucrose 0.02 gm 1h | 2X 10² | 2X 10² | 4X 10² | 109 |

The reactions were carried out as described above but replacing sucrose with glucose or lactose 0.02 gm each for 3 hours. The products of these reactions were soluble in water and showed 100% anti-microbial activity against E coli when tested by the method / process described above.

### EXAMPLE 12

### CHITOSAN COMPOSITION MADE BY USING PHOSPHORUS ACID AND CARRYING FORMALDEHYDE RESIDUES

Chitosan [50 - 70 DDA, 60 mesh] 5 gm is taken in a round bottom flask and 100 ml methanol (99%) is added to it. Any other grade of chitosan can be used here. The suspension is mixed well by stirring at 100 rpm for 30 min. The mixture is heated to 60°C. Phosphorus acid (H₃PO₃) in different proportions ranging from 0.5 g to 15 gm is dissolved in 20 ml of water and added drop-wise to this mixture by stirring for 1 hour. Formaldehyde 5 ml is then added in drop-wise manner. The temperature is maintained between 60 - 65°C and preferably at 63°C for 2 - 6 hours after the addition is complete. The suspension is allowed to cool and filtered and the solid is air dried. The free flowing powder is formed by breaking the loose lumps mechanically and sieved using 100 mesh. No lumping was observed. The free flowing powder is then further dried in hot air oven at 70°C for 3 - 6 hours.

Solubility of the free flowing particles is checked by adding 100 mg powder slowly to 100 ml distilled water (pH 6.5) over 5 min. The mixture is stirred continuously during addition and further for 10 more min. and then the solubility is checked visibly. The solubility is also checked at pH 8, 9, and 10 adjusted using dilute NaOH, or Tris buffer.

The products formed using chitosan and phosphorus acid in 1:1 and 1:2 ratio showed solubility in the broad range of pH (7 -10), forming slightly turbid to clear solutions.

These solutions showed varying degree of anti-microbial activity. The antimicrobial studies were conducted using *Pseudomonas aeruginosa* NCIM 2200 (ATCC 9027) capable of growing at a broad pH range. The overnight culture grown in the nutrient broth was inoculated (100 micro L) again in nutrient broth (10 ml) of varying pH (see the table) without any addition (control) and with chitin-phosphorus acid product. The cultures were incubated on shaker at 25°C for 8 h, and the growth was recorded as optical density (OD) of the broth at 600 nm. Low optical density (than control) indicates less growth, and some anti-microbial activity. No growth - zero OD - means 100% inhibition of microbes.

**Table 5: Inhibition of Pseudomonas at pH 8.5**

| | | C:P1:1 | C:P 1:2 |
|---|---|---|---|
| pH 7 | | | |
| Control | 1.74 | | |
| 0.01% | | 1.56 | 1.53 |
| 0.05% | | 0 | 0 |
| 0.10% | | 0 | 0 |
| pH 8 | | | |
| Control | 1.73 | | |
| 0.01% | | 1.39 | 0 |
| 0.05% | | 0 | 0 |
| 0.10% | | 0 | 0 |
| pH 9 | | | |
| Control | 1.69 | | |
| 0.01% | | 1.39 | 1.68 |
| 0.05% | | 0 | 0 |
| 0.10% | | 0 | 0 |

When banana are stored for a long time the crown of the bunch starts drying. The stem which joins the banana finger to crown is called pedicel. It starts wilting.

Wilting of pedicel is a best indicator of the drying. Less wilting means preservation of freshness.. The test was conducted on 1000 kg banana each by the standard method (control) dipping in 0.1% of our product, spray of our product. This was repeated on 2 farms (total 6000 kg banana). Very low percentage of pedicel wilting by dipping as well as spraying of the test composition shows its good efficacy in delaying in drying and ultimately on delaying ageing and senescence and improving storage capability.

The results are given in the below Table 6.

**Table 6: Effectiveness of c:p 1:1 product in fruit preservation**

| % Pedicel wilting / farm using product of Example12 | | |
|---|---|---|
| Farm number | Farm no. 1 | Farm no. 2 |
| Dipping | 2.4 | 1.9 |
| Spray | 3.2 | 2.8 |
| Control | 2.7 | 2.4 |

By dipping method significant improvement in the wilting (weight loss, or preservation of freshness) was observed.

### EXAMPLE 13

### APPLICATION OF THE PRESERVATIVE COMPOSITION

Solutions were prepared from 10 gram of chitosan and its product described in example 1, 2, 4 and 10 by dissolving chitosan in 2% acetic acid and all other derivatives in water to make a volume of 1000 ml solution in a vessel of 15 L capacity. 3 kg tomato or 2 kg banana were dipped in the solution for 15 sec. When the material was taken out from the container, excess solution was allowed to drain in the same container for 1 min. This dipping action was repeated with next five batches of fresh fruit. Thus a total of 15 kg tomato or 10 kg banana was dipped in 10 L solution.

Then the remaining solution in the vessel was accurately measured and loss of liquid was recorded as difference in the initial and final volume. This was divided by the total amount of fruit dipped in it, giving the consumption of solution per kg of the fruit dipped in. The results of this experiment are given in table 7

**Table 7:**

| Product | Consumption in ml per Kg tomato | Consumption in ml per kg banana |
|---|---|---|
| Chitosan | 12 | 19 |
| Chitosan: Lactic acid 1:0.4 as per example 1 | 9 | 10 |
| Chitosan: Succinic anhydride 1:0.1 per example 2 | 8 | 8 |
| Product of example 4 | 3 | 5 |
| Product of example 10 | 3 | 3 |

Fifth composition showed three to four times less or lesser consumption per kg of banana and tomato than the solution of chitosan in acetic acid.

### EXAMPLE 14

### ANTIMICROBIAL ACTIVITY OF CHIT0SAN COMPOSITIONS

Chitosan was dissolved in 2% acetic acid and its products described above were dissolved in water. 300 mg of the product was used to make 10 ml solution. *E.coli* NCIM 2065 and *Bacillus subtilis* NCIM 2063 cultures were grown overnight (10⁹ cells/ml density) diluted 100 folds with sterile liquid broth and used for antimicrobial assay. The assay was set by mixing diluted cultures with stock solution of chitosan or product as follows:

| | | | | |
|---|---|---|---|---|
| Culture vol. In ml | 7 | 8.5 | 9.7 | 9.9 |
| Chitosan/ product vol. in ml | 3 | 1.5 | 0.3 | 0.1 |
| Concentration of product (%) | 1 | 0.5 | 0.1 | 0.033 |

The mixture was incubated at 37°C for 20 min. The total viable count of the mixture was determined. This when compared with the initial count of the culture gives us the microbicidal effectiveness of the product. The data for *E coli* and *Bacillus subtilis* is given in table 8 and 9.

**Table 8: Initial count of E coli was 4X 10⁷**

| Product/ concentration | 1 % | 0.5% | 0.1% | 0.033% |
|---|---|---|---|---|
| Chitosan | 2X 10⁷ | 4X 10⁷ | 4X 10⁷ | 4X 10⁷ |
| Chitosan: Lactic acid 1:0.4 | 6X 10⁵ | 1X 10⁶ | 2X 10⁷ | 2X 10⁷ |
| Chitosan: Succinic anhydride 1:0.1 | 8X 10⁶ | 2X 10⁷ | 2X 10⁷ | 2X 10⁷ |
| Product of example 4 | 9X 10⁵ | 2X 10⁶ | 9X 10⁶ | 3X 10⁷ |
| Product of example 9 | 3X10⁷ | 9X 10⁶ | 3 X 10⁶ | 7X 10⁵ |
| Product of example 10 | 2X10³ | 2X10³ | 4X10³ | 1X10⁴ |

**Table 9: initial count of Bacillus subtilis 3X 10⁷**

| Product/ concentration | 1 % | 0.5% | 0.1% | 0.033% |
|---|---|---|---|---|
| Chitosan | 3X 10⁷ | 3X 10⁷ | 4X 10⁷ | 4X 10⁷ |
| Chitosan: Lactic acid 1:0.4 | 4X 10⁵ | 3X 10⁶ | 1X 10⁷ | 7X 10⁶ |
| Chitosan: Succinic anhydride 1:0.1 | 3X 10⁶ | 2X 10⁶ | 8X 10⁷ | 6X 10⁷ |
| Product of example 4 | 7X 10⁵ | 1X 10⁶ | 5X 10⁶ | 1X 10⁶ |
| Product of example 10 | 1X10⁴ | 2X10⁴ | 4X10⁵ | 1X10⁵ |

The mixture (0.1%) was incubated in refrigerator (at temperature 4 - 10°C) up to 7 weeks. Increase in the turbidity was visually detected and scored as +. No increase in the turbidity (-) indicates microbiostatic activity.

**Table 10: Microbiostatic activity against E. coli (NCIM 2065) and B. subtilis (NCIM 2063)**

| Product/ microbial culture Weeks | *E coli* | | | *B subtilis* | | |
|---|---|---|---|---|---|---|
| | 3 | 5 | 7 | 3 | 5 | 7 |
| Chitosan | + | + | + | + | + | + |
| Chitosan: Lactic acid 1:0.4 | - | + | + | - | - | + |
| Chitosan: Succinic anhydride 1:0.1 | + | + | + | - | + | + |
| Product of example 4 | - | - | + | - | - | + |
| Product of example 9 | - | - | - | - | - | + |

Fifth composition showed high antimicrobial as well as microbiostatic activity as compared to chitosan solution in acetic acid and Fisrt, Second and Fourth Composition.

### EXAMPLE 15

### EFFECT OF PRESERVATIVE COMPOSITIONS ON STORAGE STABILITY

When chitosan solution, chitosan processed by example . 6 and 7 were used to coat banana and strawberry fruits - 100 fruits for each set, the lowest number of microbial spoilages (22 and 9 for the respective fruits) were observed after 5 days of storage at room temperature and 25 for chitosan processed by example 6 method / process.

### EXAMPLE 16

### IMPROVEMENT IN THE SHELF LIFE BY PRODUCT OF EXAMPLE 10 (Fifth Composition)

Freshly harvested (<30 h of harvesting) green banana are treated using 0.1% solution of the product from example 10 in water. The treatment can be by dipping of banana in the said solution for nominally 10 - 15 sec., or by direct spray of the solution on the banana with a suitable device so as to make the surface of banana wet, or by passing the banana through a closed chamber which is saturated with the fog of the said solution (indirect fogging). The treated banana are air dried. These banana are stored at room temperature (20 - 32°C and a relative humidity of 40 - 60%. The banana from the same lot which are identical to the treated banana are kept as 'untreated control'. Both treated and untreated banana are stored under identical conditions for up to 15 days. The treated banana maintained green color, and weight where as the untreated banana lost weight and developed discoloration. The photograph of treated and untreated banana taken on 12^{th} day, shows the visual differences. The treated banana had better physiological condition as tested chemically by determination of their total solids (BRIX contents), reducing sugars, and weight loss. The data is shown in table 11 and figure 1.

**Table11: Results of the quantitative tests conducted on treated and untreated control banana on 6^{th} day of the storage.**

| Parameter | Treated | Control |
|---|---|---|
| Weight loss | 2.6 | 8.7 |
| BRIX | 22-24 | 20-22 |
| RS | 800 - 1100 | 300 - 850 |

Photograph of the untreated (left) and treated (right) banana on 12^{th} day of the storage is shown in Figure 1.

Further to the storage for 8 - 15 days, both the treated and untreated control banana were treated with ethylene. The treated banana ripened normally, whereas the untreated control banana either became hard and did not ripen, or turned black and soft. Both the types of banana from the untreated lot were not acceptable for consumption.

### EXAMPLE 17

### POSITIVE PHYSIOLOGICAL CHANGES AND MICROBIO-STATIC ACTIVITY:

The whole tomato and cut apple were treated with the product from example 10, exactly as described in example 14 for banana. The treated tomato retained its weight, texture and freshness for 30 days as against untreated control tomato, which wrinkled in 12 - 15 days resulting in weight loss. Physiological changes in the treated and control tomatoes were monitored on 7^{th} day of storage using MRI scanning technique.

The fleshy skin of the treted tomato was more uniform in its color density. The fleshy skin of the untreated control tomato has dark patches that are indicative of uneven and more drying.

The MRI scan of the representative tomatoes is included in the Figure 2

The pieces of cut apple were packed in polythene bag or transparent plastic container and stored in refrigerator at temperature of 4 - 9°C. The cut apple from control started becoming brown from day 2 and turned soft by Day 3, where as the treated cut apple retained its color, taste, and hard texture for 6 days. The photograph (Figure 3) shows the difference in the appearance of treated and untreated cut apple on day 4.

When treated and untreated cut apple pieces were packed in transparent plastic container and stored as described above for up to 25 days. The untreated cut apple developed fungal growth [black patches in the photograph in Figure 4], whereas the treated cut apple did not develop any fungal or other microbial growth. The results are depicted in the following photograph.

### EXAMPLE 18

### CHITOSAN COMPOSITION USING PHOSPHORUS ACID AND HYDROCHLORIC ACID

Chitosan [50 - 70 DDA, 100 mesh] 750 gm is taken in a round bottom flask and 15000 ml methanol (99%) is added to it. Any other grade of chitosan can be used here. The suspension is mixed well by stirring at 100 rpm for 30 min. The mixture is heated to 60°C. Formaldehyde 750 ml is then added over nominal 1-2 min.

Phosphorus acid (H₃PO₃) 750 gm is dissolved in 7500 ml of water and added drop-wise to this mixture by stirring for 1 hour. The temperature is maintained between 60 - 65°C and preferably at 63°C for 6 hours after the addition is complete. The suspension is allowed to cool and filtered and the solid is air dried. The free flowing powder - small clumps are formed. They are broken mechanically and sieved using 100 mesh. The free flowing powder is then further dried in hot air oven at 70°C for 3 - 6 hours. Solubility of the free flowing particles is checked by adding 100 mg powder slowly to 100 ml distilled water (pH 6.5) over 5 min. The mixture is stirred continuously during addition and further for 10 more min. and then the solubility is checked visibly. The solubility is also checked at pH 8, 9, and 10 adjusted using dilute NaOH, or Tris buffer.

The product dissolved in water, but precipitated out when the pH was raised to 7.0. The dry powder obtained in this reaction was resuspended in 20 volumes of 90% methanol, and 2 volumes of concentrated HCl were added. The mixture was stirred for one hour at room temperature. The powder was filtered out, washed with 20 vol 90% methanol 3 times and dried as described above. The resultant product was soluble from pH 3 to 12.

The products of all other examples - particularly example 10, 12 when treated with HCl as described in this example did not alter the maximum pH of solubility.

### EXAMPLE 19

### USE OF WATER SOLUBLE CHITOSAN AS CARRIER.

1 gm of water soluble chitosan as derived by example 10, was dissolved in 100 ml water. To 9 ml of this solution 1 ml of copper sulfate solution (1%) was added. This was hand mixed for 2 min. Then 1 ml liquid ammonia was added. This resulted in precipitation of chitosan. Along with chitosan the bound copper sulfate is also precipitated. Copper sulfate is freely soluble in liquid ammonia and the resulting solution is deep blue in color. Estimation of copper is done by colorimetry, and using a standard graph 0.02 - 0.1%. From this the chitosan bound copper was estimated to be 90%.

## Claims

1. A process for producing a chitosan derivative by activating chitosan, by suspending the said chitosan in alcohol that contains water from 0% to maximum of about 30%, stirring with heating for a period of time to get activated chitosan or chitosan precursor and thereafter reacting the said activated chitosan or activated precursor with a reagent capable of complexing by physical forces or chemical forces to prepare a derivative.

2. A process of claim 1 wherein:
a. the said derivative has a property that comprises an antimicrobial, or a microbiostatic, or an antitranspirant, or an antifungal, a fruit preservative or a microbiostatic, or shelf life improving or a combination thereof,
b. the said physical forces include adsorption and the said chemical forces include a covalent bond.

3. A process of claim 1 wherein the said derivative is prepared by subjecting the said chitosan suspended in the said alcohol to at least one of the following steps:
a. adding water to about 20% of volume of alcohol and heating the said mixture to about 70°C, adding Succinic anhydride to this mixture,
i. stirring for 1 hour or for a period of time enough for reaction at a temperature between to about 60 - 70°C and preferably at about 65°C to get a Second Composition in suspension,
ii. and either (I) recovering the said composition with or without drying, or (II) subjecting to reacting further with a reagent to get a further derivative of the said Second Composition,
b. adding water about 20% of volume of alcohol and heating the said mixture to about 70°C, adding succinic anhydride at a rate of about 10% by weight of the raw material to this mixture,
i. stirring for 1 hour or for a period of time enough for reaction maintaining a temperature to about 60 - 70°C and preferably at about 65°C to get a succinic derivative in suspension,
ii. allowing the suspension to cool to about 30°C, subjecting the said succinic derivative to further reaction with lactic or another acid about 10% to 20% of the dry weight of the succinic derivative, stirring the mixture for about 1 hour, filtering and drying the solids to get a Fourth Composition in suspension,
iii. and either (I) recovering the said composition with or without drying, or (II) subjecting to reacting further with a reagent to get a further derivative of the said Fourth Composition, which comprises a coarse granular material that is soluble in water,
c. adding water about 20% of volume of alcohol and, heating and maintaining the said mixture to about 70°C, adding Succinic anhydride to this mixture,
i. stirring for 1 hour or for a period of time enough for reaction maintaining the temperature between 60 to about - 70°C and preferably at about 65°C for a period of time,
ii. allowing the suspension to cool to get a succinic derivative, subjecting the said succinic derivative to further reaction with Lactic or another acid about 10% weight of the dry weight of the succinic derivative, per 5 gram dry weight of succinic derivative used adding enzyme for deaceylation about 4000 I.U, stirring the mixture for a period of time, preferably for 7 hours, further adding a chitinolytic enzyme, preferably around 500 I.U. per 5 g dry weight of succinic derivative, continuing stirring for a period of time, preferably for 1 hour, allowing the suspension to cool to get a Fifth Composition in suspension,
iii. and either (I) recovering the said composition with or without drying, or (II) subjecting to reacting further with a reagent to get a further derivative of the said Fifth Composition,
d. adding water about 20% of volume of alcohol and, heating the mixture to about 60°C to about 70°C, adding Succinic anhydride to this mixture,
i. stirring for 1 hour or for a period of time enough for reaction maintaining the temperature between 60 - 70°C and preferably at around 65°C for a period of time,
ii. allowing the suspension to cool to get a succinic derivative, subjecting the said succinic derivative to further reaction with Lactic or another acid about 10% of the dry weight of the succinic derivative, and per 5 gram dry weight of succinic derivative used adding enzyme for deacetylation about 4000 I.U, stirring the mixture for a period of time, preferably for 7 hours, further adding a chitinolytic enzyme 500 I.U. per 5 gram dry weight of succinic derivative used and continuing stirring for a period of time, preferably for 1 hour, allowing the suspension to cool, to get a Fifth Composition in suspension, recovering the said Fifth Composition as solid powder,
iii. suspending the dry powder of the said Fifth Composition in absolute alcohol, mixing the suspension well by stirring at 100 rpm for 30 min. and heating and maintaining the temperature throughout the process to 60°C, adding sugar dissolved in water in about 10% volume to volume of the alcohol, stirring the mixture for about 15 minutes or for a period of time enough for mixing, adding formaldehyde dropwise over about 30 minutes or over a period of time required for gradual addition with constant stirring maintaining temperature to around 60°C for about 1 to 6 hours or for a period until the reaction is complete,
iv. thereafter allowing the reaction mixture to cool down to room temperature,
v. recovering the product, washing the solids with aqueous methanol or another aqueous polar alcohol and recovering a Sixth Composition in suspension,
vi. and either (I) recovering the said composition with or without drying, or (II) subjecting to reacting further with a reagent to get a further derivative of the said Sixth Composition that may be washed with 100 ml 90% methanol and air dried,
e. suspending chitosan in alcohol preferably in 20 volumes, mixing well by stirring to achieve activation of chitosan, preferably at 100 rpm for 30 min, heating the said mixture to about 60°C , adding Phosphorus acid (H₃PO₃) dissolved in water and adding drop-wise to this mixture by stirring, adding formaldehyde about equal in weight to the dry weight of activated chitosan or its derivative in drop-wise manner maintaining the temperature between about 60 - 65°C, preferably at 63°C, for a period of time preferably to 2 - 6 hours after the addition is complete, allowing the suspension to cool and recovering the Seventh Composition in suspension, and either (I) recovering the said composition with or without drying, or (II) subjecting to reacting further with a reagent to get a further derivative of the said Seventh Composition; the said Seventh Composition may be soluble in the broad range of pH 7 to 10 giving slightly turbid to clear solutions,
f. suspending chitosan in alcohol preferably in 20 volumes, mixing well by stirring to achieve activation of chitosan, preferably at 100 rpm for 30 min, heating the mixture to 60°C, adding formaldehyde 750 ml over a period of few minutes, adding and about 10% of inorganic acid or Phosphorus acid (H₃PO₃) dissolved in water and added drop-wise to this mixture by stirring for about one hour or over a period of time required for effective mixing, maintaining the temperature between 60 - 65°C and preferably at 63°C for about six hours or for a period of time required for completion of the reaction, allowing the suspension to cool after the addition is complete and recovering the Eighth Composition in suspension,
i. and either (I) recovering the said composition with or without drying, or (II) subjecting to reacting further with a reagent to get a further derivative of the said Eighth Composition,
g. suspending chitosan in alcohol preferably in 20 volumes,mixing well by stirring to achieve activation of chitosan, preferably at 100 rpm for 30 min, heating the mixture to 60°C, adding formaldehyde over a period of few minutes, adding about 10% inorganic acid or Phosphorus acid (H₃PO₃) dissolved in water and added drop-wise to this mixture by stirring for about 1 hour or over a period of time required for effective mixing, maintaining the temperature between 60 - 65°C and preferably at 63°C for 6 hours or for a period of time required for completion of reaction after the addition is complete, allowing the suspension to cool, recovering the suspension as dry powder, resuspending in 20 volumes of 90% methanol, and 2 volumes of concentrated HCl or any other organic or inorganic acid, stirring the mixture at room temperature for one hour or for a period of time required for good mixing, filtering out, washing with 20 vol 90% methanol and drying to recover Ninth Composition,
h. suspending chitosan in alcohol preferably in 20 volumes, mixing well by stirring to achieve activation of chitosan, preferably at 100 rpm for 30 min, heating the said mixture to70°C,
i. adding Succinic anhydride in different proportions ranging from 15 g to 0.1 gm to this mixture, stirring for one hour or for a period of time required for good mixing, maintaining the temperature between 60 - 70°C and preferably at around 65°C for 1 hour or for a period of time required for the reaction, allowing the suspension to cool to get a succinic derivative,
ii. adding Lactic or another acid about 10% of the dry weight of the succinic derivative, and per 5 gram dry weight of succinic derivative used adding enzyme for deacetylation about 4000 I.U, stirring the mixture for a period of time, preferably for 7 hours, adding further chitinolytic enzyme 500 I.U. per 5 gram dry weight of succinic derivative used and continuing stirring for a period of time, preferably for 1 hour, allowing the suspension to cool to get solid Fifth Composition,
iii. recovering and air drying the Fifth Composition,
iv. dissolving the Fifth Composition to make a 1% solution in water, adding an aqueous solution of copper sulfate or another charged molecule, capable of adsorbing on the Fifth Composition in alkaline pH, as a solution,
v. the said solution of copper sulfate or the charged molecule that may be 1% with respect to water,
vi. the said solution of copper sulfate or the charged molecule being taken in a small quantity, about 1% of the volume of water taken for dissolving Fifth Composition and
vii. mixing for few minutes,
viii. adding liquid ammonia to precipitate chitosan with the bound copper sulfate or another charged particle to get a derivative of Tenth Composition.

4. A chitosan derivative produced by activating chitosan, by suspending the said chitosan in alcohol that contains water from 0% to maximum of about 30%, stirring with heating for a period of time to get activated chitosan or chitosan precursor and thereafter reacting the said activated chitosan or activated precursor with a reagent capable of complexing by physical forces or chemical forces to prepare a derivative.

5. The chitosan derivative of claim 4 wherein the said derivative has a property that comprises an antimicrobial, or a microbiostatic, or an antitranspirant, or an antifungal, or a fruit preservative or a combination thereof.

6. The chitosan derivative of claim 4 wherein the said derivative is prepared by the process of claim 3.

7. The composition comprising at least one of the compositions of claim 4 or 5 or 6 as an essential ingredient.

8. The composition of claim 7 comprising a powder of or a solution of at least one of the said compositions in water in an effective concentration for a desired action that comprises:
a. preservation of on fruits, vegetable, ingestible foods and feeds,
b. delayed ripening, delayed ageing and delayed senescence of natural products including fruits, vegetable and flowers.

9. The composition of claim 8 comprising at least one of the following:
a. the said composition comprises at least any one of the said Second composition, and Fourth Composition to the said Tenth Composition or a mixture thereof,
b. the said effective concentration is 0.1 %,
c. the acetic acid is 2%.

10. A process of improving shelf life of fruits comprising a treatment of dipping, spraying, fogging or another process of external or internal application or applications to cut or processed fruits and processed natural products of a composition comprising one or more of a composition of claim 4 or 5 or 6 as an essential ingredient.

11. The process of claim 10 wherein the said treatment is for slowing down ripening and/or microbial spoilage.

12. The process of claim 10 wherein the said fruit is banana, mango, apple, strawberry, orange, lime.

13. The process of claim 1 wherein :
a. the said alcohol is methanol or any other polar alcohol,
b. the said stirring is done for 30 min, preferably at 100 revolution per minute.

14. The process of claim 3 step c), step d) and step h) wherein the said chitinolytic enzyme having proteolytic as well as chitinolytic action comprises an enzyme having at least one of the following properties: a molecular weight of 50 kilo daltons (KD) on SDS-PAGE electrophoresis, an optimum chitinolytic activity at pH 2 and an optimum chitinolytic activity at temperature of 80⁰C.

15. The process of claim 14 comprising the said chitinolytic enzyme isolated from *Aspergillus niger* MTCC 5572 or mutants derived from the same.

16. The process of claim 3 step c), step d) and step h) comprising the said deacetylating enzyme being isolated from *Bacillus sp* MTCC 5571 or mutants derived from the same.

17. The process of claim 3 step c), step d) and step h) wherein the said deacetylating enzyme is a xylan acyl transferase prepared from *Bacillus sp* MTCC 5571 or mutants derived from the same or any other bacterium or any other microorganism capable of producing the enzyme having deacetylating activity.

18. The process of claim 1 wherein a thermostable chitinolytic enzyme having a proteolytic as well as chitinolytic activity, a proteo-chitinase, is used for making a hydrolysed derivative.

19. The process of claim 18 wherein the said proteo-chitinase is prepared from *Aspergillus niger* MTCC 5572 or mutants derived from the same or any other fungus or any other microorganism capable of producing a proteo-chitinolytic activity.

20. The process of claim 19 wherein the said chitinolytic enzyme has a molecular weight on SDS_PAGE of 50 Kilo Daltons, optimum chitinolytic activity at pH 2 and at 80°C.

## Patentansprüche

1. Verfahren zur Herstellung eines Chitosanderivats durch Aktivieren von Chitosan, durch Suspergieren des Chitosans in Alkohol, der 0 % bis maximal circa 30 % Wasser enthält, Rühren unter Erhitzen für einen Zeitraum zur Gewinnung von aktiviertem Chitosan oder einer aktivierten Chitosan-Vorstufe und anschließendes Reagieren des aktivierten Chitosans oder der aktivierten Vorstufe mit einem Reagenz, das zur Komplexbildung durch physische Kräfte oder chemische Kräfte geeignet ist, um ein Derivat herzustellen.

2. Verfahren nach Anspruch 1, wobei:
a. das Derivat eine Eigenschaft aufweist, die eine antimikrobielle oder eine mikrobiostatische oder eine schweißhemmende oder eine antimykotische, eine fruchtkonservierende oder eine mikrobiostatische oder eine die Haltbarkeit verbessernde Eigenschaft oder eine Kombination derselben aufweist,
b. die physischen Kräfte Adsorption und die chemischen Kräfte eine kovalente Bindung umfassen.

3. Verfahren nach Anspruch 1, wobei das Derivat durch Anwenden mindestens eines der folgenden Schritte auf das in dem Alkohol suspergierte Chitosan hergestellt wird:
a. Zugeben von Wasser zu circa 20 Vol.-% Alkohol und Erhitzen des Gemischs auf circa 70 °C, Zugeben von Bernsteinsäureanhydrid zu dem Gemisch,
i. Rühren für 1 Stunde oder für einen für eine Reaktion ausreichenden Zeitraum bei einer Temperatur zwischen circa 60 °C und 70 °C und vorzugsweise bei circa 65 °C zur Gewinnung einer Zweiten Zusammensetzung in Suspension,
ii. und entweder (I) Rückgewinnen der Zusammensetzung mit oder ohne Trocknen oder (II) Beaufschlagen mit einer weiteren Reaktion mit einem Reagenz zur Gewinnung eines weiteren Derivats der Zweiten Zusammensetzung,
b. Zugeben von Wasser zu circa 20 Vol.-% Alkohol und Erhitzen des Gemischs auf circa 70 °C, Zugeben von Bernsteinsäureanhydrid in einem Verhältnis von circa 10 Gew.-% des Rohmaterials zu dem Gemisch,
i. Rühren für 1 Stunde oder für einen für eine Reaktion ausreichenden Zeitraum unter Halten einer Temperatur zwischen circa 60 °C und 70 °C und vorzugsweise bei circa 65 °C zur Gewinnung eines Bernsteinsäurederivats in Suspension,
ii. Abkühlenlassen der Suspension auf circa 30 °C, Beaufschlagen des Bernsteinsäurederivats mit einer weiteren Reaktion mit Milchsäure oder einer anderen Säure in einer Menge von circa 10 % bis 20 % des Trockengewichts des Bernsteinsäurederivats, Rühren des Gemischs für circa 1 Stunde, Filtern und Trocknen der Feststoffe zur Gewinnung einer Vierten Zusammensetzung in Suspension,
iii. und entweder (I) Rückgewinnen der Zusammensetzung mit oder ohne Trocknen oder (II) Beaufschlagen mit einer weiteren Reaktion mit einem Reagenz zur Gewinnung eines weiteren Derivats der Vierten Zusammensetzung, welche ein in Wasser lösliches grobkörniges Material umfasst,
c. Zugeben von Wasser zu circa 20 Vol.-% des Alkohols und Erhitzen und Halten des Gemischs bei circa 70 °C, Zugeben von Bernsteinsäureanhydrid zu dem Gemisch,
i. Rühren für 1 Stunde oder für einen für eine Reaktion ausreichenden Zeitraum unter Halten der Temperatur zwischen 60 °C und circa 70 °C und vorzugsweise bei circa 65 °C für einen Zeitraum,
ii. Abkühlenlassen der Suspension zur Gewinnung eines Bernsteinsäurederivats, Beaufschlagen des Bernsteinsäurederivats mit einer weiteren Reaktion mit Milchsäure oder einer anderen Säure in einer Menge von circa 10 Gew.-% des Trockengewichts des Bernsteinsäurederivats, Zugeben von circa 4000 IE eines Enzyms zur Deacetylierung pro 5 Gramm Trockengewicht des verwendeten Bernsteinsäurederivats, Rühren des Gemischs für einen Zeitraum, vorzugsweise für 7 Stunden, weiterhin Zugeben von vorzugsweise 500 IE eines chitinolytischen Enzyms pro 5 Gramm Trockengewicht des Bernsteinsäurederivats, Weiterrühren für einen Zeitraum, vorzugweise für 1 Stunde, Abkühlenlassen der Suspension zur Gewinnung einer Fünften Zusammensetzung in Suspension,
iii. und entweder (I) Rückgewinnen der Zusammensetzung mit oder ohne Trocknen oder (II) Beaufschlagen mit einer weiteren Reaktion mit einem Reagenz zur Gewinnung eines weiteren Derivats der Fünften Zusammensetzung,
d. Zugeben von Wasser zu circa 20 Vol.-% Alkohol und Erhitzen des Gemischs auf circa 60 °C bis circa 70 °C, Zugeben von Bernsteinsäureanhydrid zu dem Gemisch,
i. Rühren für 1 Stunde oder für einen für eine Reaktion ausreichenden Zeitraum unter Halten der Temperatur zwischen 60 °C und 70 °C und vorzugsweise bei circa 65 °C für einen Zeitraum,
ii. Abkühlenlassen der Suspension zur Gewinnung eines Bernsteinsäurederivats, Beaufschlagen des Bernsteinsäurederivats mit einer weiteren Reaktion mit Milchsäure oder einer anderen Säure in einer Menge von circa 10 % des Trockengewichts des Bernsteinsäurederivats, Zugeben von circa 4000 IE eines Enzyms zur Deacetylierung pro 5 Gramm Trockengewicht des verwendeten Bernsteinsäurederivats, Rühren des Gemischs für einen Zeitraum, vorzugsweise für 7 Stunden, weiterhin Zugeben von 500 IE eines chitinolytischen Enzyms pro 5 Gramm Trockengewicht des verwendeten Bernsteinsäurederivats und Weiterrühren für einen Zeitraum, vorzugweise für 1 Stunde, Abkühlenlassen der Suspension zur Gewinnung einer Fünften Zusammensetzung in Suspension, Rückgewinnen der Fünften Zusammensetzung als festes Pulver,
iii. Suspergieren des trockenen Pulvers der Fünften Zusammensetzung in reinem Alkohol, gutes Durchmischen der Suspension bei 100 U/min für 30 min und Erhitzen und Halten der Temperatur während des Prozesses bei 60 °C, Zugeben von in Wasser gelöstem Zucker zu etwa 10 Vol.-% des Gesamtvolumens, Rühren des Gemischs für circa 15 min oder für einen für ein Durchmischen ausreichenden Zeitraum, tropfenweises Zugeben von Formaldehyd für circa 30 min oder für einen zur graduellen Zugabe ausreichenden Zeitraum bei stetem Rühren unter Halten der Temperatur bei circa 60 °C für circa 1 bis 6 Stunden oder für einen Zeitraum bis zum Abschluss der Reaktion,
iv. anschließend Abkühlenlassen des Reaktionsgemischs auf Raumtemperatur,
v. Rückgewinnen des Produkts, Waschen der Feststoffe mit wässrigem Methanol oder einem anderen wässrigen polaren Alkohol und Rückgewinnen einer Sechsten Zusammensetzung in Suspension,
vi. und entweder (I) Rückgewinnen der Zusammensetzung mit oder ohne Trocknen oder (II) Beaufschlagen mit einer weiteren Reaktion mit einem Reagenz zur Gewinnung eines weiteren Derivats der Sechsten Zusammensetzung, welche mit 100 ml 90-prozentigem Methanol gewaschen und luftgetrocknet werden kann,
e. Suspergieren von Chitosan in Alkohol in vorzugsweise 20 Volumen-%, gutes Durchmischen durch Rühren bei vorzugsweise 100 U/min für 30 min zum Erreichen der Aktivierung von Chitosan, Erhitzen des Gemischs auf circa 60 °C, Zugeben von in Wasser gelöster und dem Gemisch unter Rühren tropfenweise zugegebener Phosphonsäure (H₃PO₃), tropfenweises Zugeben von Formaldehyd im circa gleichen Gewicht wie dem Trockengewicht von aktiviertem Chitosan oder dessen Derivat unter Halten der Temperatur zwischen circa 60 °C und 65 °C, vorzugsweise bei 63 °C, für einen Zeitraum von vorzugsweise 2 bis 6 Stunden nach beendeter Zugabe, Abkühlenlassen der Suspension und Rückgewinnen der Siebten Zusammensetzung in Suspension und entweder (I) Rückgewinnen der Zusammensetzung mit oder ohne Trocknen oder (II) Beaufschlagen mit einer weiteren Reaktion mit einem Reagenz zur Gewinnung eines weiteren Derivats der Siebten Zusammensetzung; wobei die Siebte Zusammensetzung im breiten Bereich von pH 7 bis 10 löslich sein kann und leicht trübe bis klare Lösungen ergibt,
f. Suspergieren von Chitosan in Alkohol in vorzugsweise 20 Volumen-%, gutes Durchmischen durch Rühren bei vorzugsweise 100 U/min für 30 min zum Erreichen der Aktivierung von Chitosan, Erhitzen des Gemischs auf circa 60 °C, Zugeben von 750 ml Formaldehyd über einen Zeitraum von wenigen Minuten, Zugeben von 10 % in Wasser gelöster und dem Gemisch tropfenweise zugegebener anorganischer Säure oder Phosphonsäure (H₃PO₃) durch Rühren für circa eine Stunde oder über einen zur wirksamen Durchmischung benötigten Zeitraum, Halten der Temperatur zwischen 60 °C und 65 °C und vorzugsweise bei 63 °C, für circa 6 Stunden oder für einen zum Abschluss der Reaktion benötigten Zeitraum, Abkühlenlassen der Suspension nach beendeter Zugabe und Rückgewinnen der Achten Zusammensetzung in Suspension,
i. und entweder (I) Rückgewinnen der Komposition mit oder ohne Trocknen oder (II) Beaufschlagen mit einer weiteren Reaktion mit einem Reagenz zur Gewinnung eines weiteren Derivats der Achten Zusammensetzung,
g. Suspergieren von Chitosan in Alkohol in vorzugsweise 20 Volumen-%, gutes Durchmischen durch Rühren bei vorzugsweise 100 U/min für 30 min zum Erreichen der Aktivierung von Chitosan, Erhitzen des Gemischs auf circa 60 °C, Zugeben von Formaldehyd über einen Zeitraum von wenigen Minuten, Zugeben von 10 % in Wasser gelöster und dem Gemisch tropfenweise zugegebener anorganischer Säure oder Phosphonsäure (H₃PO₃) durch Rühren für circa eine Stunde oder über einen zur wirksamen Durchmischung benötigten Zeitraum, Halten der Temperatur zwischen 60 °C und 65 °C und vorzugsweise bei 63 °C für 6 Stunden oder für einen zum Abschluss der Reaktion benötigten Zeitraum nach beendeter Zugabe, Abkühlenlassen der Suspension, Rückgewinnen der Suspension als trockenes Pulver, erneutes Suspergieren in 20 Volumen-% von 90-%igem Methanol und 2 Volumen-% konzentrierter HCl oder einer anderen organischen oder anorganischen Säure, Rühren des Gemischs bei Raumtemperatur für eine Stunde oder für einen zur guten Durchmischung benötigten Zeitraum, Ausfiltern, Waschen mit 20 Vol.-% von 90-%igem Methanol und Trockenen zur Rückgewinnung einer Neunten Zusammensetzung,
h. Suspergieren von Chitosan in Alkohol in vorzugsweise 20 Volumen-%, gutes Durchmischen durch Rühren bei vorzugsweise 100 U/min für 30 min zum Erreichen der Aktivierung von Chitosan, Erhitzen des Gemischs auf 70 °C,
i. Zugeben von Bernsteinsäureanhydrid in verschiedenen Anteilen von 15 g bis 0,1 gm zu diesem Gemisch, Rühren für eine Stunde oder für einen zur guten Durchmischung benötigten Zeitraum, Halten der Temperatur zwischen 60 °C und 70 °C und vorzugweise bei circa 65 °C für eine Stunde oder für einen für die Reaktion benötigten Zeitraum, Abkühlenlassen der Suspension zur Gewinnung eines Bernsteinsäurederivats,
ii. Zugeben von Milchsäure oder einer anderen Säure in einer Menge von circa 10 % des Trockengewichts des Bernsteinsäurederivats und Zugeben von circa 4000 IE eines Enzyms zur Deacetylierung pro 5 Gramm Trockengewicht des verwendeten Bernsteinsäurederivats, Rühren des Gemischs für einen Zeitraum, vorzugsweise für 7 Stunden, Zugeben von weiteren 500 IE chitinolytischen Enzyms pro 5 Gramm Trockengewicht des verwendeten Bernsteinsäurederivats und Weiterrühren für einen Zeitraum, vorzugsweise für 1 Stunde, Abkühlenlassen der Suspension zur Gewinnung der festen Fünften Zusammensetzung,
iii. Rückgewinnen und Lufttrocknen der Fünften Zusammensetzung,
iv. Auflösen der Fünften Zusammensetzung zur Herstellung einer 1-%igen Lösung in Wasser, Zugeben einer wässrigen Lösung aus Kupfersulfat oder einem anderen zur Adsorption an der Fünften Zusammensetzung in alkalischem pH geeigneten geladenen Molekül als Lösung,
v. Zugeben der Lösung aus Kupfersulfat oder dem geladenen Molekül, welches bezogen auf Wasser 1 % betragen kann,
vi. wobei die Lösung aus Kupfersulfat oder dem geladenen Molekül in einer geringen Menge von circa 1 % des Volumens des zum Auflösen der Fünften Zusammensetzung verwendeten Wassers verwendet wird, und
vii. Durchmischen für wenige Minuten,
viii. Zugeben von flüssigem Ammoniak zum Ausfällen von Chitosan mit dem gebundenen Kupfersulfat oder einem anderen geladenen Partikel zur Gewinnung eines Derivats einer Zehnten Zusammensetzung.

4. Chitosanderivat, hergestellt durch Aktivierung von Chitosan, Suspergieren des Chitosans in Alkohol, der 0 % bis maximal 30 % Wasser enthält, Rühren unter Erhitzen für einen Zeitraum zur Gewinnung von aktiviertem Chitosan oder einer aktivierten Chitosan-Vorstufe und anschließendes Reagieren des aktivierten Chitosans oder der aktivierten Vorstufe mit einem zur Komplexbildung durch physische Kräfte oder chemische Kräfte geeigneten Reagenz zur Herstellung eines Derivats.

5. Chitosanderivat nach Anspruch 4, wobei das Derivat eine Eigenschaft aufweist, die eine antimikrobielle oder eine mikrobiostatische oder eine schweißhemmende oder eine antimykotische oder eine fruchtkonservierende Eigenschaft oder eine Kombination derselben umfasst.

6. Chitosanderivat nach Anspruch 4, wobei das Derivat nach dem Verfahren nach Anspruch 3 hergestellt ist.

7. Zusammensetzung, umfassend mindestens eine der Zusammensetzungen nach Anspruch 4 oder 5 oder 6 als wesentlichen Bestandteil.

8. Zusammensetzung nach Anspruch 7, umfassend ein Pulver oder eine Lösung von mindestens einer der Zusammensetzungen in Wasser in einer für eine gewünschte Wirkung wirksamen Konzentration, wobei die Wirkung Folgendes umfasst:
a. Haltbarmachung von Obst, Gemüse, Nahrungs- und Futtermitteln,
b. verzögerte Reifung, verzögerte Alterung und verzögerte Seneszenz von Naturprodukten einschließlich Obst, Gemüse und Blumen.

9. Zusammensetzung nach Anspruch 8, umfassend mindestens eines der folgenden Merkmale:
a. die Zusammensetzung umfasst die Zweite Zusammensetzung und zumindest eine der Vierten bis Zehnten Zusammensetzungen oder ein Gemisch derselben,
b. die wirksame Konzentration beträgt 0,1 %,
c. die Essigsäure ist 2-%ig.

10. Verfahren zur Verbesserung der Haltbarkeit von Obst, umfassend eine Behandlung durch Tauchen, Sprühen, Nebeln oder einen anderen Prozess zur externen oder internen Anwendung oder Anwendungen von Zusammensetzungen, die eine oder mehrere der Zusammensetzungen nach Anspruch 4 oder 5 oder 6 als wesentlichen Bestandteil umfasst, auf geschnittenes oder verarbeitetes Obst und verarbeitete Naturprodukte.

11. Verfahren nach Anspruch 10, wobei die Behandlung zur Verlangsamung der Reifung und/oder des mikrobiellen Verderbs dient.

12. Das Verfahren nach Anspruch 10, wobei es sich bei dem Obst um Banane, Mango, Apfel, Erdbeere, Orange, Limette handelt.

13. Verfahren nach Anspruch 1, wobei:
a. der Alkohol Methanol oder ein anderer polarer Alkohol ist,
b. das Rühren für 30 min bei vorzugweise 100 U/min erfolgt.

14. Verfahren nach Anspruch 3, Schritt c), Schritt d) und Schritt h), wobei das chitinolytische Enzym mit proteolytischer sowie chitinolytischer Wirkung ein Enzym umfasst, das mindestens eine der folgenden Eigenschaften aufweist: ein Molekulargewicht von 50 Kilodalton (KD) gemäß SDS-PAGE-Elektrophorese, eine optimale chitinolytische Aktivität bei pH 2 und eine optimale chitinolytische Aktivität bei einer Temperatur von 80 °C.

15. Verfahren nach Anspruch 14, umfassend das aus *Aspergillus niger* MTCC 5572 oder aus davon abgeleiteten Mutanten isolierte chitinolytische Enzym.

16. Verfahren nach Anspruch 3, Schritt c), Schritt d) und Schritt h), umfassend das aus *Bacillus sp* MTCC 5571 oder aus davon abgeleiteten Mutanten isolierte deacetylierende Enzym.

17. Verfahren nach Anspruch 3, Schritt c), Schritt d) und Schritt h), wobei das deacetylierende Enzym eine Xylan-Acyltransferase ist, die aus *Bacillus sp* MTCC 5571 oder aus davon abgeleiteten Mutanten oder aus einem anderen Bakterium oder einem anderen Mikroorganismus hergestellt ist, das/der zur Produktion des Enzyms mit deacetylierender Aktivität in der Lage ist.

18. Verfahren nach Anspruch 1, wobei ein thermostabiles chitinolytisches Enzym mit proteolytischer sowie chitinolytischer Aktivität - eine Proteochitinase - zur Herstellung eines hydrolysierten Derivats verwendet wird.

19. Verfahren nach Anspruch 18, wobei die Proteochitinase aus *Aspergillus niger* MTCC 5572 oder aus davon abgeleiteten Mutanten oder aus einem anderen Pilz oder einem anderen Mikroorganismus hergestellt wird, der zur Produktion einer proteochitinolytischen Aktivität in der Lage ist.

20. Verfahren nach Anspruch 19, wobei das chitinolytische Enzym gemäß SDS-PAGE-Elektrophorese ein Molekulargewicht von 50 Kilodalton und eine optimale chitinolytische Aktivität bei pH 2 und bei 80 °C aufweist.

## Revendications

1. Procédé de production d'un dérivé de chitosane par l'activation du chitosane en suspendant le chitosane dans de l'alcool qui contient de l'eau de 0 % à un maximum de 30 %, en remuant tout en chauffant pour une période de temps afin d'obtenir du chitosane activé ou un précurseur de chitosane et en réagissant ci-après ledit chitosane activé ou ledit précurseur activé avec un réactif capable de complexer par des forces physiques ou des forces chimiques afin de préparer un dérivé.

2. Procédé selon la revendication 1, dans lequel :
a. ledit dérivé a une propriété qui comprend une propriété antimicrobienne ou microbiostatique ou antitranspirante ou antifongique ou conservatrice de fruits ou microbiostatique ou améliorant la durée de conservation ou une combinaison de ces propriétés,
b. les forces physiques comprennent l'adsorption et les forces chimiques comprennent une liaison covalente.

3. Procédé selon la revendication 1, dans lequel le dérivé est préparé en soumettant le chitosane suspendu dans ledit alcool à au moins une des étapes suivantes :
a. ajouter de l'eau à environ 20 % en volume d'alcool et chauffer ledit mélange à environ 70 °C, ajouter de l'anhydride succinique à ce mélange,
i. remuer pour 1 heure ou pour une période de temps suffisante pour la réaction à une température entre environ 60 °C et 70 °C et de préférence à environ 65 °C afin d'obtenir une Deuxième Composition en suspension,
ii. et soit (I) récupérer ladite composition avec ou sans séchage, soit (II) soumettre à une autre réaction avec un réactif afin d'obtenir un autre dérivé de ladite Deuxième Composition,
b. ajouter de l'eau à environ 20 % en volume d'alcool et chauffer ledit mélange à environ 70 °C, ajouter de l'anhydride succinique à un taux d'environ 10 % en poids de la matière première à ce mélange,
i. remuer pour 1 heure ou pour une période de temps suffisante pour la réaction tout en maintenant une température d'environ 60 °C à 70 °C et de préférence à environ 65 °C afin d'obtenir un dérivé succinique en suspension,
ii. laisser refroidir la suspension à environ 30 °C, soumettre ledit dérivé succinique à une autre réaction avec de l'acide lactique ou un autre acide d'environ 10 % à 20 % du poids sec du dérivé succinique, remuer le mélange pour environ 1 heure, filtrer et sécher les solides afin d'obtenir une Quatrième Composition en suspension,
iii. et soit (I) récupérer ladite composition avec ou sans séchage, soit (II) soumettre à une autre réaction avec un réactif afin d'obtenir un autre dérivé de ladite Quatrième Composition, qui comprend un gros matériau granulaire qui est soluble dans l'eau,
c. ajouter de l'eau à environ 20 % en volume de l'alcool et chauffer et maintenir ledit mélange à environ 70 °C, ajouter de l'anhydride succinique à ce mélange,
i. remuer pour 1 heure ou pour une période de temps suffisante pour la réaction tout en maintenant à une température entre environ 60 °C et 70 °C et de préférence à environ 65 °C pour une période de temps,
ii. laisser refroidir la suspension afin d'obtenir un dérivé succinique, soumettre ledit dérivé succinique à une autre réaction avec de l'acide lactique ou un autre acide d'environ 10 % en poids du poids sec du dérivé succinique, ajouter environ 4000 UI d'une enzyme pour la désacétylation par 5 grammes de poids sec du dérivé succinique utilisé, remuer le mélange pour une période de temps, de préférence pour 7 heures, ajouter en outre une enzyme chitinolytique, de préférence environ 500 UI par 5 grammes de poids sec du dérivé succinique, continuer à remuer pour une période de temps, de préférence pour 1 heure, laisser refroidir la suspension afin d'obtenir une Cinquième Composition en suspension,
iii. et soit (I) récupérer ladite composition avec ou sans séchage, soit (II) soumettre à une autre réaction avec un réactif afin d'obtenir un autre dérivé de ladite Cinquième Composition,
d. ajouter de l'eau à environ 20 % en volume d'alcool et chauffer le mélange à environ 60 °C à environ 70 °C, ajouter de l'anhydride succinique à ce mélange,
i. remuer pour 1 heure ou pour une période de temps suffisante pour la réaction tout en maintenant la température entre 60 °C et 70 °C et de préférence à environ 65 °C pour une période de temps,
ii. laisser refroidir la suspension afin d'obtenir un dérivé succinique, soumettre ledit dérivé succinique à une autre réaction avec de l'acide lactique ou un autre acide d'environ 10 % du poids sec du dérivé succinique, et ajouter environ 4000 UI d'une enzyme pour la désacétylation par 5 grammes de poids sec du dérivé succinique utilisé, remuer le mélange pour une période de temps, de préférence pour 7 heures, ajouter en outre une enzyme chitinolytique, de préférence environ 500 UI par 5 grammes de poids sec de dérivé succinique utilisé et continuer à remuer pour une période de temps, de préférence pour 1 heure, laisser refroidir la suspension afin d'obtenir une Cinquième Composition en suspension, récupérer ladite Cinquième Composition sous la forme de poudre solide,
iii. suspendre la poudre sèche de la Cinquième Composition dans de l'alcool absolu, bien mélanger la suspension en remuant à 100 tr/min pour 30 min et chauffer et maintenir la température à 60 °C tout au long du processus, ajouter du sucre dissout dans l'eau à environ 10 % en volume du volume total, remuer le mélange pour environ 15 minutes ou pour une période de temps suffisante pour le mélanger, ajouter du formaldéhyde goutte à goutte pendant environ 30 minutes ou pendant une période de temps nécessaire pour l'addition graduelle en remuant constamment tout en maintenant la température à environ 60 °C pour environ 1 à 6 heures ou pour une période de temps jusqu'à ce que la réaction soit terminée,
iv. ci-après laisser refroidir le mélange de réaction à la température ambiante,
v. récupérer le produit, laver les solides avec du méthanol aqueux ou un autre alcool polaire aqueux et récupérer une Sixième Composition en suspension,
vi. et soit (I) récupérer ladite composition avec ou sans séchage, soit (II) soumettre à une autre réaction avec un réactif afin d'obtenir un autre dérivé de la Sixième Composition qui peut être lavée avec 100 ml de 90 % méthanol et séchée à l'air,
e. suspendre du chitosane dans de l'alcool de préférence à 20 % en volume, bien mélanger en remuant afin d'atteindre l'activation du chitosane, de préférence à 100 tr/min pour 30 min, chauffer ledit mélange à environ 60 °C, ajouter de l'acide phosphoreux (H₃PO₃) dissout dans l'eau en l'ajoutant goutte à goutte à ce mélange en remuant, ajouter goutte à goutte du formaldéhyde en poids environ égal au poids sec du chitosane activé ou de son dérivé tout en maintenant la température entre environ 60 °C et 65 °C, de préférence à 63 °C, pour une période de temps de préférablement 2 à 6 heures après la terminaison de l'addition, laisser refroidir la suspension et récupérer la Septième Composition en suspension, et soit (I) récupérer ladite composition avec ou sans séchage, soit (II) soumettre à une autre réaction avec un réactif afin d'obtenir un autre dérivé de ladite Septième Composition ; ladite Septième Composition pouvant être soluble dans la vaste gamme de pH 7 à 10, rendant des solutions légèrement troubles à claires,
f. suspendre du chitosane dans de l'alcool de préférence à 20 % en volume, bien mélanger en remuant afin d'atteindre l'activation du chitosane, de préférence à 100 tr/min pour 30 min, chauffer le mélange à 60 °C, ajouter 750 ml de formaldéhyde pendant une période de quelques minutes, ajouter environ 10 % d'acide inorganique ou d'acide phosphoreux (H₃PO₃) dissout dans l'eau et ajouté goutte à goutte à ce mélange en remuant pour environ une heure ou pendant une période de temps nécessaire pour le mélange efficace, maintenir la température entre 60 °C et 65 °C et de préférence à 63 °C pour environ six heures ou pour une période de temps nécessaire pour la terminaison de la réaction, laisser refroidir la suspension après la terminaison de l'addition et récupérer la Huitième Composition en suspension,
i. et soit (I) récupérer ladite composition avec ou sans séchage, soit (II) soumettre à une autre réaction avec un réactif afin d'obtenir un autre dérivé de ladite Huitième Composition,
g. suspendre du chitosane dans de l'alcool de préférence à 20 % en volume, bien mélanger en remuant afin d'atteindre l'activation du chitosane, de préférence à 100 tr/min pour 30 min, chauffer le mélange à 60 °C, ajouter du formaldéhyde pendant une période de quelques minutes, ajouter environ 10 % d'acide inorganique ou d'acide phosphoreux (H₃PO₃) dissout dans l'eau et ajouté goutte à goutte à ce mélange en remuant pour environ 1 heure ou pendant une période de temps nécessaire pour le mélange efficace, maintenir la température entre 60 °C et 65 °C et de préférence à 63 °C pour 6 heures ou pour une période de temps nécessaire pour la terminaison de la réaction après la terminaison de l'addition, laisser refroidir la suspension, récupérer la suspension sous la forme de poudre sèche, resuspendre dans 20 volumes de 90 % méthanol et 2 volumes de HCl concentré ou d'un autre acide organique ou inorganique, remuer le mélange à température ambiante pour une heure ou pour une période de temps nécessaire pour le bien mélanger, filtrer, laver avec 20 % en volume de 90 % méthanol et sécher afin de récupérer une Neuvième Composition,
h. suspendre du chitosane dans de l'alcool de préférence en 20 volumes, bien mélanger en remuant afin d'atteindre l'activation du chitosane, de préférence à 100 tr/min pour 30 min, chauffer le mélange à 70 °C,
i. ajouter de l'anhydride succinique en proportions différentes allant de 15 g à 0,1 gm à ce mélange, remuer pour une heure ou pour une période de temps nécessaire pour le bien mélanger, maintenir la température entre 60 °C et 70 °C et de préférence à environ 65 °C pour 1 heure ou pour une période de temps nécessaire pour la réaction, laisser refroidir la suspension afin d'obtenir un dérivé succinique,
ii. ajouter de l'acide lactique ou un autre acide d'environ 10 % du poids sec du dérivé succinique, ajouter environ 4000 UI d'une enzyme pour la désacétylation par 5 grammes de poids sec du dérivé succinique utilisé, remuer le mélange pour une période de temps, de préférence pour 7 heures, ajouter en outre une enzyme chitinolytique, de préférence environ 500 UI par 5 grammes de poids sec du dérivé succinique utilisé, et continuer à remuer pour une période de temps, de préférence pour 1 heure, laisser refroidir la suspension afin d'obtenir la Cinquième Composition solide,
iii. récupérer et sécher la Cinquième Composition à l'air,
iv. dissoudre la Cinquième Composition afin de produire une solution de 1 % dans l'eau, ajouter une solution aqueuse du sulfate de cuivre ou d'une autre molécule chargée qui est capable d'adsorber sur la Cinquième Composition en pH alcalin sous la forme d'une solution,
v. ajouter ladite solution du sulfate de cuivre ou de la molécule chargée, qui peut être 1 % par rapport à l'eau,
vi. ladite solution du sulfate de cuivre ou la molécule chargée étant prise en une petite quantité, environ 1 % du volume de l'eau prise pour dissoudre la Cinquième Composition et
vii. mélanger pour quelques minutes,
viii. ajouter de l'ammoniaque liquide afin de précipiter du chitosane avec le sulfate de cuivre lié ou une autre particule chargée liée afin d'obtenir un dérivé d'une Dixième Composition.

4. Dérivé de chitosane, produit en activant du chitosane, en suspendant ledit chitosane dans de l'alcool qui contient de l'eau de 0 % à un maximum de 30 %, en remuant tout en chauffant pour une période de temps afin d'obtenir du chitosane activé ou un précurseur de chitosane et en réagissant ci-après ledit chitosane activé ou ledit précurseur activé avec un réactif capable de complexer par des forces physiques ou des forces chimiques afin de préparer un dérivé.

5. Dérivé de chitosane selon la revendication 4, dans lequel ledit dérivé a une propriété qui comprend une propriété antimicrobienne ou microbiostatique ou antitranspirante ou antifongique ou conservatrice de fruits ou une combinaison de ces propriétés.

6. Dérivé de chitosane selon la revendication 4, dans lequel ledit dérivé est préparé par le procédé selon la revendication 3.

7. Composition, comprenant au moins une des compositions selon la revendication 4 ou 5 ou 6 comme ingrédient essentiel.

8. Composition selon la revendication 7, comprenant une poudre ou une solution d'au moins une desdites compositions dans l'eau en une concentration efficace pour une action désirée, qui comprend :
a. la conservation de fruits, de légumes, de comestibles et de matières fourragères,
b. la maturité retardée, le vieillissement retardé et la sénescence retardée de produits naturels, y compris les fruits, les légumes et les fleurs.

9. Composition selon la revendication 8, comprenant au moins un des suivants:
a. la composition comprend au moins une de ladite Deuxième Composition et la Quatrième Composition à ladite Dixième Composition ou un mélange de ces compositions,
b. ladite concentration efficace est 0,1 %,
c. l'acide acétique est 2 %.

10. Procédé pour l'amélioration de la durée de conservation de fruits comprenant un traitement de trempage, pulvérisation, brumisation ou un autre procédé d'application ou d'applications externes ou internes d'une composition comprenant une ou plusieurs d'une composition selon la revendication 4 ou 5 ou 6 comme ingrédient essentiel à des fruits coupés ou transformés et des produits naturels transformés.

11. Procédé selon la revendication 10, dans lequel ledit traitement sert à ralentir la maturité et/ou la détérioration microbienne.

12. Procédé selon la revendication 10, dans lequel ledit fruit est la banane, la mangue, la pomme, la fraise, l'orange, la lime.

13. Procédé selon la revendication 1, dans lequel :
a. ledit alcool est le méthanol ou un autre alcool polaire,
b. on remue pour 30 min, de préférence à 100 tours par minute.

14. Procédé selon la revendication 3, étape c), étape d) et étape h), dans lequel ladite enzyme chitinolytique ayant une action protéolytique ainsi que chitinolytique comprend une enzyme ayant au moins une des propriétés suivantes : un poids moléculaire de 50 kilodaltons (KD) selon l'électrophorèse SDS-PAGE, une activité chitinolytique optimale à pH 2 et une activité chitinolytique optimale à une température de 80 °C.

15. Procédé selon la revendication 14, comprenant ladite enzyme chitinolytique isolée à partir d'*Aspergillus niger* MTCC 5572 ou à partir de mutants dérivés de celui-ci.

16. Procédé selon la revendication 3, étape c), étape d) et étape h), comprenant ladite enzyme désacétylique étant isolée à partir de *Bacillus sp* MTCC 5571 ou à partir de mutants dérivés de celui-ci.

17. Procédé selon la revendication 3, étape c), étape d) et étape h), dans lequel ladite enzyme désacétylique est une acétyle xylane transférase préparée à partir de *Bacillus sp* MTCC 5571 ou à partir de mutants dérivés de celui-ci ou à partir d'une autre bactérie ou d'un autre microorganisme capable de produire l'enzyme ayant l'activité désacétylique.

18. Procédé selon la revendication 1, dans lequel une enzyme chitinolytique thermostable ayant une activité protéolytique ainsi que chitinolytique, une protéo-chitinase, est utilisée pour produire un dérivé hydrolysé.

19. Procédé selon la revendication 18, dans lequel ladite protéo-chitinase est préparée à partir d'*Aspergillus niger* MTCC 5572 ou à partir de mutants dérivés de celui-ci ou de tout autre champignon ou de tout autre microorganisme capable de produire une activité protéo-chitinolytique.

20. Procédé selon la revendication 19, dans lequel ledit enzyme chitinolytique a un poids moléculaire de 50 kilodaltons selon SDS_PAGE et une activité chitinolytique optimale à pH 2 et à 80 °C.
